# EUROPEAN PATENT APPLICATION

(11) **EP 1 780 264 A2**
(43) Date of publication of application: **02.05.2007**
(21) Application number: 06026923.0
(22) Date of filing: 17.12.2002
(51) Int. Cl.: C12N 5/04, C12N 15/11

(54) **Nucleic acid nematicides**

(30) Priority: 17.12.2001 GB 0130199
(62) Divisional of application: 02788132.5
(71) Applicant: THE UNIVERSITY OF LEEDS, Leeds, LS2 9JT (GB)
(72) Inventor: Atkinson, Howard, Leeds LS2 9JJ (GB); McPherson, Michael, Leeds LS2 9JJ (GB); Urwin, Peter, Leeds LS2 9JJ (GB)
(74) Representative: Harrison Goddard Foote

(57) **Abstract**

The invention is the identification of double stranded RNA nematicides to nematode genes and a screening assay for the identification of agents, typically dsRNA's, with nematicidal activity.

SEQUENCE LISTING

<110> The University of Leeds

<120> Nucleic Acid Nematicides

<130> P101440WO

<140> PCT/GB2002/005727
<141> 2003-12-17

<150> 0130199.3
<151> 2001-12-17

<160> 19

<170> PatentIn version 3.1

<210> 1
<211> 4494
<212> DNA
<213> Caenorhabditis elegans

<400> 1
atgcgctcaa aacatgtgct gtacatagct atactgttca gttcaatttt tggagggaaa 60

ggaatccaac aaaatgagga atttcaaaga tacgacggat ggtacaacaa tctggcgaat 120

agtgaatggg gttctgctgg aagtcggctg catagagatg cacgttccta ctactcagac 180

ggtgtatatt cagtgaataa ctcacttccg tccgcccgtg aactctccga tatactattc 240

aaaggagagt ccggtatacc taatacaaga ggatgcacga ctttattggc atttttcagt 300

caagtagttg ctcatgaaat aatgcaatca aatggagtat cctgtccact agagacactt 360

aaaattcaag tacccctatg tgataatgta tttgataaag aatgtgaggg aaagacagaa 420

atcccattta cacgtgccaa atacgataaa gcaactggaa atgggctcaa ctcacctcga 480

gaacaaatca atgaacggac ttcatggatt gatggatcat tcatctatgg taccacccag 540

ccatgggtgt cctcattaag atctttcaaa caagggcggt tggctgaagg tgtacctgga 600

tatccaccac ttaacaaccc acatattcca ttgaataacc ccgctccgcc acaagtacat 660

cgattgatga gtccagatag attatttatg ttgggagact cgcgtgtgaa tgagaatcca 720

ggtcttctct catttggtct gatcctcttc cgttggcata actacaatgc aaatcaaatc 780

catcgagaac atcctgactg gacagacgaa caaatcttcc aggcagcacg tcgtttggtg 840

attgcatcta tgcagaagat tattgcatat gactttgttc cagggctgtt aggtgaagac 900

gttcgtttgt caaactacac caaatacatg ccacatgttc cacctggaat ctcgcatgct 960

tttggagcag ccgccttcag gttccctcac tcaattgtgc caccagcaat gcttctgaga 1020

aaacgaggaa ataaatgtga attccggacg gaagttggtg gatatcctgc attgagattg 1080

tgccagaatt ggtggaatgc gcaggatatt gtaaaggagt acagtgtgga tgagattatt 1140

cttggaatgg caagccagat agctgaacga gatgataaca tagtagttga agatcttcgt 1200

gattacatct tcggaccaat gcatttctct cgtttggatg ttgttgcttc atcaataatg 1260

agaggaaggg acaatggagt accaccgtat aatgaattga gaagaacatt cggacttgcg 1320

ccaaagacat gggagacaat gaatgaagac ttttacaaga agcatactgc aaaggtggag 1380

aagttgaaag agttgtatgg aggcaatatt ttatatttgg atgcttatgt aggaggaatg 1440

ctggaaggag gtgaaaatgg gcctggagag ttgttcaaag aaatcataaa ggatcaattc 1500

acccgtattc gtgatggaga tagattctgg tttgagaata aattgaatgg attattcact 1560

gatgaagaag ttcaaatgat tcatagtatt acacttcgag atattatcaa agcaaccacc 1620

gatatcgatg agacaatgct tcagaaggat gtattcttct tcaaggaagg tgacccgtgc 1680

ccgcaaccat tccaagtgaa cacaactgga cttgaaccat gtgttccatt tatgcaatca 1740

acttattgga ctgataatga caccacttat gttttcaccc taattggatt agcatgtgtg 1800

ccattaattt gctatggaat tggccgatac ttggttaatc gtcgcattgc tattggccac 1860

aacagtgctt gtgacagcct aactactgac tttgcaaatg atgattgtgg cgcgaaggga 1920

gatatttatg gtgtaaatgc tttggaatgg cttcaagaag agtacatacg acaggtcagg 1980

atagaaatag aaaacaccac gttggcagta aagaagccac gcggtggaat ccttcgaaaa 2040

attcgttttg aaactggaca gaagattgag ttattccact ctatgccgaa tccatcagca 2100

atgcacggac catttgtact tctgtctcaa aagaataatc atcatttggt gataagattg 2160

tcgtctgata gagatttatc taaatttttg gatcaaatta gacaggcggc tagtggaatc 2220

aatgcagagg ttatcataaa ggatgaggag aattctattc tcttatccca agcaatcaca 2280

aaagaacgcc gtcaagaccg actggacctg ttcttccgtg aagcctacgc aaaagcattc 2340

aatgatagtg aacttcaaga ttcggaaact tcatttgact catcaaatga tgatatatta 2400

aatgagacaa tatctcgtga ggaactggca agtgcaatgg gaatgaaagc gaataatgag 2460

tttgtgaaga gaatgttcgc gatgattgca aaacataatg aggattcgct cagtttcaat 2520

gagtttttga cagtcttgag agagtttgtt aatgctcctc aaaagcaaaa actgcaaact 2580

ctattcaaaa tgtgtgattt ggagggaaag aacaaggtac tccgaaagga tctcgcggaa 2640

ctcgtcaagt ccctcaatca aaccgctgga gttcacatta ctgaaagtgt gcagcttcga 2700

ttattcaatg aagtgttgca ctatgcagga gtgagcaatg atgccaagta cctgacttac 2760

gacgatttca atgctctgtt ctcggatata cctgacaagc aaccagttgg actgccgttc 2820

aatcgaaaga actatcagcc aagtattgga gaaacatctt ctctgaactc atttgccgtc 2880

gtggatcgat ccatcaacag ttcagcaccg ctaactttga tccacaaagt ttcagcgttc 2940

ttggagacct atcgccaaca cgttttcatt gtcttctgct ttgttgccat caatcttgtt 3000

cttttcttcg aacggttttg gcattatcgt tacatggcgg aaaacaggga tctccgacga 3060

gtaatgggag ctggaatcgc tattactcgt ggtgccgcgg gagccttgtc attttgcatg 3120

gcgttgatat tgctgacagt ttgtagaaac ataatcacac ttcttcgaga gacagtcatt 3180

gcgcagtata ttccatttga ctcggctatt gcgttccaca agatcgttgc gctctttgcg 3240

gctttctggg ccactcttca caccgttgga cattgtgtca atttctatca cgttggaact 3300

caaagtcaag aaggtcttgc ttgtctcttt caggaagcat tctttggatc caacttcctt 3360

ccttcaatca gttactggtt cttcagcaca attacaggtc tgacaggaat tgcattggtc 3420

gctgtcatgt gcatcattta tgttttcgcg ttaccatgtt tcattaagag agcttatcac 3480

gcattccggc tcacacatct tctcaatatt gccttttacg cacttactct tcttcatggg 3540

cttccaaagt tgttggattc tcccaaattt ggctactacg ttgttggtcc catcgtgtta 3600

tttgtaattg atcgcataat tggtttgatg caatattaca aaaaattaga aattgtaaac 3660

gcagaaatcc ttccatcaga tattatatac atcgagtacc gtcgtccaag agagtttaaa 3720

tataaatcag gacaatgggt tactgtatca tcaccatcaa tatcatgtac ctttaatgaa 3780

tctcacgcat tctcgattgc ctcaagtcca caggatgaga atatgaagtt gtatataaaa 3840

gcagttggac catggacatg gaagttgaga agcgaattga taagatcatt gaatacagga 3900

tcgccatttc cattaatcca tatgaaagga ccatatggtg atggtaacca agaatggatg 3960

gattatgaag ttgcaataat ggttggagca ggaatcggag tgactccata tgcatcgaca 4020

cttgttgatc ttgtacaacg aacatcaagt gactcatttc acagagttcg ttgccgtaaa 4080

gtatatttcc tatgggtgtg ctcaactcac aagaactatg aatggtttgt ggatgtgctc 4140

aagaacgtgg aagaccaagc aaggtcggga attttggaga cacatatctt tgtcactcag 4200

acgttccaca agtttgattt gagaactact atgctttaca tttgcgagaa gcacttccgt 4260

gccaccaact caggaatttc aatgtttact ggtctccacg ctaagaacca tttcggacgg 4320

cccaacttca aagctttctt ccaatttatt cagagtgaac ataaggagca atccaaaatc 4380

ggagtgttca gttgtggacc tgtaaacttg aatgaaagta tagctgaagg atgtgcagat 4440

gccaaccgac aacgagatgc tccttcattt gcacatcgct ttgaaacgtt ctaa 4494

<210> 2
<211> 1260
<212> DNA
<213> Caenorhabditis elegans

<400> 2
tacggcgggg ggtataataa ttttgcaaat ccacaattgg gaagtgttcg cagtcgactg 60

catagagacg gggctagcag ttatcaagat ggtgtttaca gattagattc atctttgcct 120

tcagctagag ttatttctca attaatgttt aaaggagaac ctggcattcc tagcagacgc 180

aatctgacaa caatgttcgc ctttttcagt caggtcattg cctatgaaat aatgcaatca 240

acacaaaaca gttgcccttt ggaaatgcat aaaattcctg tcgaacgttg tgatccaata 300

tttgacaagg attgtgaagg aaaaactgat attcctttta ctagagcaaa atatgacaaa 360

gggactggac atggattaaa ttcccctcga gaacaaatta atgaaagaac tagttggatt 420

gatgcttcct ttctctatag tactcaagag ccgtgggttg cagcattacg ttcattcgag 480

aatggcactc ttcttgaagg cccaatgcct ggctacccct cctttaatga tccgcacatc 540

cctttaatta atcctccacc tccacaaatt catagactaa tgaatcctga aagacttttc 600

attttgggtg atccaagaat taacgaaaat cccggtcttc tcagttttgg actaattctc 660

tttcgttggc ataatattca agctttgaga ttacaacagg aatttcctga atggacagat 720

gaggagcttt ttcagggtgc cagacgtttg gttattgcta ctttacaaag tattgttctc 780

tacgagtttt tgcctgtttt gttaagcatt tcaaaagaag aaataccaga atatcaaggc 840

tataatcctc atgttccacc aggaatttct cattcatttg caactacagc ctttagattt 900

ccacatactt tagtacctcc agcattatta cttagaaaaa gaaacggaaa ttgtgaattt 960

agaaaagagg ttggaggctt tccagctctt cgactttgtc agaattggtg gaatgcacag 1020

gatattgtac gtgaatattc tgtcgatgaa attgtcttag gaatggcttc tcaaatagcg 1080

gaggatgagg atcatatagt tgttgaagac ttacgtgatt tcattttcgg tccaatgcat 1140

tttactcgtc tagatgttgt ttccacttca attatgagag caagggataa tggattacct 1200

ggatataacc aattaagaaa ggcatataaa ttaaaaccaa atgattggac tacaataaat 1260

<210> 3
<211> 1321
<212> DNA
<213> Heterodera glycines

<400> 3
caaatatttc aattctccca attttataaa gcaagtaaaa tgtttcttct tttcttatta 60

tcaatgcttc tacttcagac aaatggttgg cgtgcccgcg agcgtgcaat tgaattggcc 120

gactcggacg aatcgatcga attgcagaac attggccaac ggaaaacgga cattcgaaca 180

ccgacagaac gaatgtctgc tcttcgtcaa atgatcgaac gcggcttttc cgattggaat 240

gcttacaaac agaagcatgg gaaagcatac gcggaccaag aagtggagaa cgaacggatg 300

ctgacttatt tgagcgccaa acagttcatt gacaagcaca acgaggcgta caaagagggc 360

aaagtgtcct tccgagtggg agagactcat attgccgacc tgcccttttc cgaataccaa 420

aagctgaacg gattccgtcg tttgatgggc gacagtttgc gccgcaatgc gtccactttt 480

ctggcgccaa tgaatgtggg cgatttgccg gaatcggtgg actggcggga caaaggatgg 540

gtgaccgaag tgaaaaacca gggaatgtgc ggctcgtgct gggcattcag tgccaccggc 600

gcattggagg gacaacacgt gcgcgacaag ggacatcttg tttcactgtc ggaacaaaat 660

ctgatcgact gctcgaagaa gtacggaaac atgggctgca acggaggcat catggacaac 720

gccttccaat acattaagga caacaaaggc atcgacaaag agacggccta cccctacaag 780

gccaagaccg gcaaaaagtg tttgttcaag cgcaacgacg tgggggcaac cgactcgggt 840

tataacgaca tagccgaagg ggacgaggag gacctgaaga tggctgttgc aacgcaaggg 900

cccgtctcag ttgccattga tgctggtcac cgttccttcc aattgtacac caacggcgtt 960

tactttgaga aggaatgcga cccggaaaat ttggaccatg gtgtgctcgt ggtgggctac 1020

ggcaccgacc caacccaagg cgactattgg attgtgaaga acagctgggg cacccgctgg 1080

ggcgagcagg gatacattcg catggcacgc aatcgcaaca acaattgcgg catcgcttcc 1140

cacgcctctt tcccattggt ctgatcggag tgaatttgtt gcccttgcgc tgattcagag 1200

acatttcatt tgattaatcg tgcaaaatga taagataatt gataatccat cagtcaatcg 1260

gtcgatttcc attttttatg ttcgcaattt tattcacata taaataaatt acttatttta 1320

a 1321

<210> 4
<211> 2066
<212> DNA
<213> Heterodera glycines

<400> 4
cccacccgaa aatgttccat ttggccgcca tcgttgcgct agcggcgctc gcactgcccc 60

tggcatcttc agacaagttc agaccatgcc tcgagggcta cgcacaatac ggcaaatact 120
gctacaaggc attcacaaac ccggacaaaa gcctgccatg gtcagacgct aacaaaatgt 180
gcaagactga aggtgccgaa ctcgcgtcag tgcacagcga aacggagcgc gaatttgtga 240
acatgctcgt ccttagggaa gcgaacaaaa tggaattcaa ctgcaaacag ccgggccgcg 300
ccttcttttg gtttggcctg aagctgaaat ggagcgaaaa gaagctggag gatgcaagtt 360
ggacggatgg aaagccggtg gactacttga gcccgaccga gtggggcgcg tacaaacggc 420
cgttgacgat ggacaacgca ggcggggccg gctacaacaa agagaacgaa aactgcgttt 480
tccttggcca atggccgcgc aaagtgaagg aatatctgtc gctggagacg gcgctggaca 540
aaagcggcgt ggatgtgctg tggatggacg tggtgtgcga gaagccggag gagtactggg 600
caccgcacgg cgccgtgtgc aaaatgcgcg ccaaggagga ggagaaggga tacggtaaag 660
acaagtacgg tgcaaaggag gaagaggagg aggaggacga agagcttcgc aagatcaaca 720
agtacgtcac catcggctcg ggcaacttca tctacgccaa caagaacacg tacatgattc 780
tcctcctgaa agacttgtac ggcaagcagt tgaaggagga gagcaggaag aaggtgtgcg 840
aattgtacaa tggcgaatat gtgaaggtca aggacgagcc gaaggaggca caagaatacc 900
tggacaaagt gctggccaag gaaaaggagc tgtactctga cgccagcgac cacatgttct 960
gccgcttcac cggcatcatc ggcgcgtgcg gcgagtcgtg ctacaaagtg ccgttgccaa 1020
tctgtgccga gggatacgcg tacttcagcg ggtactgcta caaggcgttc accaacgcgg 1080
acaaagaagt caaatattcg gaagcgcaga agatttgcaa gtcggacggg gccgaactcg 1140
tgtcgatgca ctcggagttg gagcgcgagt ttgtgaatgt tgtggcactg actgcggcga 1200
agaaatacga atacaacgcc aaagtgccgg gattggcctt ctactggacc agcatgcagt 1260
tggagtggga cgagaagaag ctaaagaacg ccagctgggc cgacatgacg ccgatggact 1320
acttgagccc gatggagtgg ggcacgaaga agcgtccggt gtacgtggac aactcgaacg 1380
gcggcgagta caacaaagaa aacgaaaact gcgtgttcat tggccaatgg ccacgcgagg 1440
gcaagcagta cctgaagttg gactcggcag tagacaagta cggtctggac gtgctgtgga 1500
gagacgaggt gtgcgagaag ccgggcgagg actgtccgcg cggcgccgtg tgtaagaaga 1560
gggctacggt gggcgaatgc tacggcacag gggcgtatct cctggacgcg gccgaggagg 1620
aggagatgaa gaagctgctc aaattcatca ctgtcggatc cggcactttt gtctatgctg 1680
aaaagaacac atacatggtc ctcctgctga aggacttgta cggcaaacag atgaaggagg 1740
aggacaggaa gaaggtgtgc gcttactaca aggcggagta cgtcactgta aaggccgagc 1800

cgaacaaagt gcaagaatat gtggacaaag tgttgtccaa gcagaagcag atgtacgcgg 1860

atgcgagcga ccacatgctg tgtaagttca gcgggacact cggcggatgc ggcgactatt 1920

gcaaaacggg atattgagaa gggacggacg gaagggaagg aacactttct cattagcatt 1980

tagtcgtcct ttgttttagt taaaatggtg cgctttttct ctctgattta ttgcataaat 2040

aaaaaatttt aaaaaaaaaa aaaaaa 2066

<210> 5
<211> 641
<212> PRT
<213> Heterodera glycines

<400> 5

Met Phe His Leu Ala Ala Ile Val Ala Leu Ala Ala Leu Ala Leu Pro
1               5                   10                  15

Leu Ala Ser Ser Asp Lys Phe Arg Pro Cys Leu Glu Gly Tyr Ala Gln
            20                  25                  30

Tyr Gly Lys Tyr Cys Tyr Lys Ala Phe Thr Asn Pro Asp Lys Ser Leu
        35                  40                  45

Pro Trp Ser Asp Ala Asn Lys Met Cys Lys Thr Glu Gly Ala Glu Leu
    50                  55                  60

Ala Ser Val His Ser Glu Thr Glu Arg Glu Phe Val Asn Met Leu Val
65                  70                  75                  80

Leu Arg Glu Ala Asn Lys Met Glu Phe Asn Cys Lys Gln Pro Gly Arg
                85                  90                  95

Ala Phe Phe Trp Phe Gly Leu Lys Leu Lys Trp Ser Glu Lys Lys Leu
            100                 105                 110

Glu Asp Ala Ser Trp Thr Asp Gly Lys Pro Val Asp Tyr Leu Ser Pro
        115                 120                 125

Thr Glu Trp Gly Ala Tyr Lys Arg Pro Leu Thr Met Asp Asn Ala Gly
    130                 135                 140

Gly Ala Gly Tyr Asn Lys Glu Asn Glu Asn Cys Val Phe Leu Gly Gln
145                 150                 155                 160

Trp Pro Arg Lys Val Lys Glu Tyr Leu Ser Leu Glu Thr Ala Leu Asp
                165                 170                 175

Lys Ser Gly Val Asp Val Leu Trp Met Asp Val Val Cys Glu Lys Pro
            180                 185                 190

Glu Glu Tyr Trp Ala Pro His Gly Ala Val Cys Lys Met Arg Ala Lys
        195                 200                 205

Glu Glu Glu Lys Gly Tyr Gly Lys Asp Lys Tyr Gly Ala Lys Glu Glu
    210                 215                 220

Glu Glu Glu Glu Asp Glu Glu Leu Arg Lys Ile Asn Lys Tyr Val Thr
225                 230                 235                 240

Ile Gly Ser Gly Asn Phe Ile Tyr Ala Asn Lys Asn Thr Tyr Met Ile
                245                 250                 255

Leu Leu Leu Lys Asp Leu Tyr Gly Lys Gln Leu Lys Glu Glu Ser Arg
            260                 265                 270

Lys Lys Val Cys Glu Leu Tyr Asn Gly Glu Tyr Val Lys Val Lys Asp
        275                 280                 285

Glu Pro Lys Glu Ala Gln Glu Tyr Leu Asp Lys Val Leu Ala Lys Glu
    290                 295                 300

Lys Glu Leu Tyr Ser Asp Ala Ser Asp His Met Phe Cys Arg Phe Thr
305                 310                 315                 320

Gly Ile Ile Gly Ala Cys Gly Glu Ser Cys Tyr Lys Val Pro Leu Pro
                325                 330                 335

Ile Cys Ala Glu Gly Tyr Ala Tyr Phe Ser Gly Tyr Cys Tyr Lys Ala
            340                 345                 350

Phe Thr Asn Ala Asp Lys Glu Val Lys Tyr Ser Glu Ala Gln Lys Ile
        355                 360                 365

Cys Lys Ser Asp Gly Ala Glu Leu Val Ser Met His Ser Glu Leu Glu
    370                 375                 380

Arg Glu Phe Val Asn Val Val Ala Leu Thr Ala Ala Lys Lys Tyr Glu
385                 390                 395                 400

Tyr Asn Ala Lys Val Pro Gly Leu Ala Phe Tyr Trp Thr Ser Met Gln
                405                 410                 415

Leu Glu Trp Asp Glu Lys Lys Leu Lys Asn Ala Ser Trp Ala Asp Met
            420                 425                 430

Thr Pro Met Asp Tyr Leu Ser Pro Met Glu Trp Gly Thr Lys Lys Arg
        435                 440                 445

Pro Val Tyr Val Asp Asn Ser Asn Gly Gly Glu Tyr Asn Lys Glu Asn
    450                 455                 460

Glu Asn Cys Val Phe Ile Gly Gln Trp Pro Arg Glu Gly Lys Gln Tyr
465                 470                 475                 480

Leu Lys Leu Asp Ser Ala Val Asp Lys Tyr Gly Leu Asp Val Leu Trp
                485                 490                 495

Arg Asp Glu Val Cys Glu Lys Pro Gly Glu Asp Cys Pro Arg Gly Ala
            500                 505                 510

Val Cys Lys Lys Arg Ala Thr Val Gly Glu Cys Tyr Gly Thr Gly Ala
        515                 520                 525

Tyr Leu Leu Asp Ala Ala Glu Glu Glu Glu Met Lys Lys Leu Leu Lys
    530                 535                 540

Phe Ile Thr Val Gly Ser Gly Thr Phe Val Tyr Ala Glu Lys Asn Thr
545                 550                 555                 560

Tyr Met Val Leu Leu Leu Lys Asp Leu Tyr Gly Lys Gln Met Lys Glu
                565                 570                 575

Glu Asp Arg Lys Lys Val Cys Ala Tyr Tyr Lys Ala Glu Tyr Val Thr
            580                 585                 590

Val Lys Ala Glu Pro Asn Lys Val Gln Glu Tyr Val Asp Lys Val Leu
        595                 600                 605

Ser Lys Gln Lys Gln Met Tyr Ala Asp Ala Ser Asp His Met Leu Cys
    610                 615                 620

Lys Phe Ser Gly Thr Leu Gly Gly Cys Gly Asp Tyr Cys Lys Thr Gly
625                 630                 635                 640

Tyr

<210>  6
<211>  1316
<212>  DNA
<213>  Globodera rostochiensis

<400>  6
cgatgacatg taggcgctct gggcgccgcc agctgctgga gctgctccgc cgggcgatga      60

catgtaggcg ctctgggcgc cgccagctgc tggagctgct ccgccgggcg atgacatgta     120

ggcgctctga tcgctcatgt ttggtgaatt gctgcgacga actgcgtaga cgagcttgct     180

gtggactgaa gttgttgctg tgttgcttct gaagattggc aatggtgatg aatgagcaaa     240

tccacttcct tatatacgct tttcaataat gaaattcgtt tcaaaaaaaa taaagagtga     300

caaacgctcc agccgaaatg tatataagcg aatggatttt ccaaattatc cacatcagca     360

atcaacaaat caacgttctc aacaatctac acatcaagcg agctcgacaa cagcatcaac     420

aacaatggcg caacttcctc cagaagacat cgcaacaatg cccgcccaga aggtcgtctt     480

caacgcaccg ttcgacaaca aggccaccta ctacgtgcgg gtaagtcagc atcgtttgac     540

cggggaacat aatctcattt aatcccttct aatttagatc atcaatcctg ggacaaagcg     600

catcggcttc gcgttcaaaa cgaccaaacc gaagcgcatc aacatgaacc ctccgaatgg     660

cgtgctcggc cccaaggagt ccgtcaacgt ggccatctcc tgcgacgcgt tcgaccccag     720

ctccgaggac accaaaggcg accgtgtgac cgtggaatgg tgcaacacac ctgatccggc     780

ggctgccgcg ttcaagctcg agtggttcca gggagacggt atggtgcgcc gcaagaactt     840

gccgatcgag tacaacgtct agacgaagat gcgccgccgc attacgacca gatgcagaag     900

ataggatccg cccccccctc caatatgtaa atgccctcat gttgtatccc cctgaacaga     960

taattttgta aaccaccata tcatcttatg tctgttcata tgtatgtaaa tgtgtaataa    1020

aatgtagctt tgattggcgc aacaaaaaaa tttgggaaaa atggttgaca cgttctgaat    1080

ttttgggcct tgctttgttt cccaattcat gtccttcccg caccaatgaa aaatgtagaa    1140

atttgttaga gtaggggcgg gcgaatgtat tgaaaacatg gtttcaattt tttgagaaaa    1200

tttttgtcat aattcctgca aaaacaggcc catagcattt agtggcctct ttagactaca 1260

aaagattgaa gccgtacaca cacacgcgat acaacggatt gttctttccc cagtta 1316

<210> 7
<211> 860
<212> DNA
<213> Globodera rostochiensis

<400> 7
tgctgttgga cgtgaagttg ttgctgtgtt gcttctgaag gctggagaca atggtgctga 60

aacaccaaat ccatttcctt ttatacactt ttcactgatg aaattcgttt caaaaaaaat 120

aaagaaattg ttacgaaacg ctccagccga aatgtatata agcgaatgga ttttccaaat 180

tatccacatc agcaatcaac aaatcaacgt tctcaacaat ctacacatca agcgagctcg 240

acaacagcat caacaacaat ggcgcaactt cctccaggag acatcgcaac aatgcccaac 300

cagaaggtcg tcttcaacgc accgttcgac aacaaggcca cctactacgt gcgggtaagt 360

tagcatcgtt tgaccgggga acataatctc atttaatccc tttcaattta ggtcatcaat 420

cctgggacaa atcgcatcgg cttcgcgttc aaaacgacca aaccgaagcg catcaacatg 480

aaccctccga atggcgtgct cggccccaag gagtccgtca acgtggccat ctcctgcgac 540

gcgttcgacc ccagctccga ggacaccaaa ggcgaccgtg tgaccgtgga atggtgcaac 600

acacctgatc cggcggctgc cgcgttcaag ctcgagtggt tccagggaga cggtatggtg 660

cgccgcaaga acttgccgat cgagtacaac gtctagacga agatgcgccg catacgacca 720

gatgcagaat atagatagga tccatccccc gattcccgca tattgtatct cccctgacgt 780

gaaaccatat atcatcatgt atgtttgttc atatgtaaat gtgtaaataa aatgttgtgg 840

tgaggggcgc gacaaaaaaa 860

<210> 8
<211> 984
<212> DNA
<213> Globodera rostochiensis

<400> 8
tgacatgtag gcgctctcat cgctcatgtt tggtgaattg ttgcggcgaa cggcgtagac 60

gagcttgctg tggactgaag ttgttgctgt gttgcttctg aagattggca atggtgtgac 120

gaatgaccaa atccatttcc ttttatacat ttttcactga tgaaattctt tacaaaaatt 180

aagaaattgt gacaacgcca aaagtatata agcgaacgga ttttccaaat tatccacatt 240

cagcaaacaa caaatcaacg ttctcaacaa tctacacatc aagcgagctc gacaacagca 300

tcaacaacaa tggcgcaact tcctccagaa gacatcgcaa caatgcccgc ccagaaggtc 360

gtcttcaacg caccgttcga caacaaggcc acctactacg tgcgggtaag ttagcatcgt 420

ttgccggggg aacataatct catttaatcc ttcctaattt agatcatcaa tcctgggaca 480

aagcgcatcg gcttcgcgtt caaaacgacc aaaccgaagc gcatcaacat gaaccctccg 540

aatggcgtgc tcggccccaa ggagtccgtc aacgtggcca tctcctgcga cgcgttcgac 600

cccagctccg aggactccaa aggcgaccgt gtgaccgtgg aatggtgcaa cacacctgat 660

ccggcggctg ccgcgttcaa gctcgagtgg ttccagggag acggtatggt gcgccgcaag 720

aacttgccga tcgagtacaa cgtctagacg aagatgcgcc gccgcattac gaccagatgc 780

agaagatagg atccccaccc cctccaatat gtaaatgccc tcatgttgta tccccctgaa 840

cagataattt tgtaaaccac catatcatct tatgcctgtt catatgtatg taatgtgtaa 900

taaaatgtag ttttgattgg cgcaacaaaa aaatttgata aaaatggttg agacgccttg 960

aattttgtgc cttgctttgt ttcc 984

<210> 9
<211> 428
<212> PRT
<213> Meloidogyne incognita

<400> 9
   Tyr Gly Gly Gly Tyr Asn Asn Phe Ala Asn Pro Gln Leu Gly Ser Val
   1 5 10 15
   Arg Ser Arg Leu His Arg Asp Gly Ala Ser Ser Tyr Gln Asp Gly Val
   20 25 30
   Tyr Arg Leu Asp Ser Ser Leu Pro Ser Ala Arg Val Ile Ser Gln Leu
   35 40 45
   Met Phe Lys Gly Glu Pro Gly Ile Pro Ser Arg Arg Asn Leu Thr Thr
   50 55 60
   Met Phe Ala Phe Phe Ser Gln Val Ile Ala Tyr Glu Ile Met Gln Ser
   65 70 75 80
   Thr Gln Asn Ser Cys Pro Leu Glu Met His Lys Ile Pro Val Glu Arg
   85 90 95
   Cys Asp Pro Ile Phe Asp Lys Asp Cys Glu Gly Lys Thr Asp Ile Pro
   100 105 110
   Phe Thr Arg Ala Lys Tyr Asp Lys Gly Thr Gly His Gly Leu Asn Ser
   115 120 125
   Pro Arg Glu Gln Ile Asn Glu Arg Thr Ser Trp Ile Asp Ala Ser Phe
   130 135 140
   Leu Tyr Ser Thr Gln Glu Pro Trp Val Ala Ala Leu Arg Ser Phe Glu
   145 150 155 160
   Asn Gly Thr Leu Leu Glu Gly Pro Met Pro Gly Tyr Pro Ser Phe Asn
   165 170 175
   Asp Pro His Ile Pro Leu Ile Asn Pro Pro Pro Pro Gln Ile His Arg
   180 185 190
   Leu Met Asn Pro Glu Arg Leu Phe Ile Leu Gly Asp Pro Arg Ile Asn
   195 200 205
   Glu Asn Pro Gly Leu Leu Ser Phe Gly Leu Ile Leu Phe Arg Trp His
   210 215 220
   Asn Ile Gln Ala Leu Arg Leu Gln Gln Glu Phe Pro Glu Trp Thr Asp
   225 230 235 240
   Glu Glu Leu Phe Gln Gly Ala Arg Arg Leu Val Ile Ala Thr Leu Gln
   245 250 255
   Ser Ile Val Leu Tyr Glu Phe Leu Pro Val Leu Leu Ser Ile Ser Lys
   260 265 270
   Glu Glu Ile Pro Glu Tyr Gln Gly Tyr Asn Pro His Val Pro Pro Gly
   275 280 285
   Ile Ser His Ser Phe Ala Thr Thr Ala Phe Arg Phe Pro His Thr Leu
   290 295 300
   Val Pro Pro Ala Leu Leu Leu Arg Lys Arg Asn Gly Asn Cys Glu Phe
   305 310 315 320
   Arg Lys Glu Val Gly Gly Phe Pro Ala Leu Arg Leu Cys Gln Asn Trp
                   325                 330                 335
   Trp Asn Ala Gln Asp Ile Val Arg Glu Tyr Ser Val Asp Glu Ile Val
               340                 345                 350
   Leu Gly Met Ala Ser Gln Ile Ala Glu Asp Glu Asp His Ile Val Val
           355                 360                 365
   Glu Asp Leu Arg Asp Phe Ile Phe Gly Pro Met His Phe Thr Arg Leu
       370                 375                 380
   Asp Val Val Ser Thr Ser Ile Met Arg Ala Arg Asp Asn Gly Leu Pro
   385                 390                 395                 400
   Gly Tyr Asn Gln Leu Arg Lys Ala Tyr Lys Leu Lys Pro Asn Asp Trp
                   405                 410                 415
   Thr Thr Ile Asn Pro Lys Leu Asn Glu Thr Asn Pro
               420                 425

<210> 10
<211> 479
<212> PRT
<213> Anopheles gambiae

<400> 10

Met Ser His Val Glu Lys Gln Arg Tyr Asp Gly Trp Tyr Asn Asn Leu
1               5                   10                  15

Ala His Pro Asp Trp Gly Ala Val Asp Asn His Leu Thr Arg Lys Ala
            20                  25                  30

Pro Ser Ala Tyr Ser Asp Gly Val Tyr Val Met Ala Gly Ser Asn Arg
        35                  40                  45

Pro Ser Pro Arg Lys Leu Ser Arg Leu Phe Met Arg Gly Thr Asp Gly
    50                  55                  60

Leu Pro Ser Met Glu Asn Arg Thr Ala Leu Leu Ala Phe Phe Gly Gln
65                  70                  75                  80

Val Val Thr Asn Glu Ile Val Met Ala Ser Glu Ser Gly Cys Pro Ile
                85                  90                  95

Glu Met His Arg Ile Glu Ile Glu Lys Cys Asp Glu Met Tyr Asp Arg
            100                 105                 110

Glu Cys Arg Gly Asp Arg Tyr Ile Pro Phe His Arg Ala Ala Tyr Asp
        115                 120                 125

Arg Asn Thr Gly Gln Ser Pro Asn Ala Pro Arg Glu Gln Ile Asn Gln
    130                 135                 140

Met Thr Ala Trp Ile Asp Gly Ser Phe Ile Tyr Ser Thr Ser Glu Ala
145                 150                 155                 160

Trp Leu Asn Ala Met Arg Ser Phe Gln Asp Gly Ala Leu Leu Thr Asp
                165                 170                 175

Lys Gln Gly Thr Met Pro Val Lys Asn Thr Met Arg Val Pro Leu Phe
            180                 185                 190

Asn Asn Pro Val Pro His Val Met Arg Met Leu Ser Pro Glu Arg Leu
        195                 200                 205

Tyr Leu Leu Gly Asp Pro Arg Thr Asn Gln Asn Pro Ala Leu Leu Ser
    210                 215                 220

Phe Ala Ile Leu Phe Leu Arg Trp His Asn Val Val Ala Lys Arg Val
225                 230                 235                 240

Arg Arg Gln His Arg Asp Trp Ser Asp Glu Glu Ile Phe Gln Arg Ala
                245                 250                 255

Arg Arg Val Val Ile Ala Ser Leu Gln Asn Ile Val Ala Tyr Glu Tyr
            260                 265                 270

Leu Pro Ala Phe Leu Asp Lys Glu Ile Pro Pro Tyr Asp Gly Tyr Lys
        275                 280                 285

Ala Asp Thr His Pro Gly Val Ser His Met Phe Gln Ala Ala Ala Phe
    290                 295                 300

Arg Phe Gly His Ser Leu Ile Pro Pro Gly Leu Phe Arg Arg Asp Gly
305                 310                 315                 320

Gln Cys Asn Phe Arg Arg Thr Asn Met Asp Phe Pro Ala Leu Arg Leu
                325                 330                 335

Cys Ser Thr Trp Trp Asn Ser Asn Asp Val Leu Asp Asn Thr Pro Val
            340                 345                 350

Glu Glu Phe Ile Met Gly Met Ala Ser Gln Ile Ala Glu Lys Glu Asp
        355                 360                 365

Pro Leu Leu Cys Ser Asp Val Arg Asp Lys Leu Phe Gly Pro Met Glu
    370                 375                 380

Phe Thr Arg Arg Asp Leu Gly Ala Leu Asn Ile Met Arg Gly Arg Asp
385                 390                 395                 400

Asn Gly Leu Pro Asp Tyr Asn Thr Ala Arg Ala Ala Tyr Arg Leu Pro
                405                 410                 415

Lys Lys Lys Ser Trp Arg Asp Ile Asn Pro Ala Val Phe Glu Arg Gln
            420                 425                 430

Pro Glu Leu Leu Asp Leu Leu Ile Lys Thr Tyr Asp Asn Gln Leu Asp
        435                 440                 445

Asn Val Asp Val Tyr Val Gly Gly Met Leu Glu Ser Asp Gly Arg Pro
    450                 455                 460

Gly Glu Leu Phe Ser Ala Val Ile Ile Asp Gln Phe Thr Arg Ile
465                 470                 475

<210> 11
<211> 480
<212> PRT
<213> Drosophila melanogaster

<400> 11

Met Tyr Ser Gln Thr Glu Lys Gln Arg Tyr Asp Gly Trp Tyr Asn Asn
1               5                   10                  15

Leu Ala His Pro Asp Trp Gly Ser Val Asp Ser His Leu Val Arg Lys
            20                  25                  30

Ala Pro Pro Ser Tyr Ser Asp Gly Val Tyr Ala Met Ala Gly Ala Asn

35                  40                  45

Arg Pro Ser Thr Arg Arg Leu Ser Arg Leu Phe Met Arg Gly Lys Asp
    50                  55                  60

Gly Leu Gly Ser Lys Phe Asn Arg Thr Ala Leu Leu Ala Phe Phe Gly
65                  70                  75                  80

Gln Leu Val Ala Asn Glu Ile Val Met Ala Ser Glu Ser Gly Cys Pro
                85                  90                  95

Ile Glu Met His Arg Ile Glu Ile Glu Lys Cys Asp Glu Met Tyr Asp
            100                 105                 110

Arg Glu Cys Arg Gly Asp Lys Tyr Ile Pro Phe His Arg Ala Ala Tyr
        115                 120                 125

Asp Arg Asp Thr Gly Gln Ser Pro Asn Ala Pro Arg Glu Gln Ile Asn
    130                 135                 140

Gln Met Thr Ala Trp Ile Asp Gly Ser Phe Ile Tyr Ser Thr Ser Glu
145                 150                 155                 160

Ala Trp Leu Asn Ala Met Arg Ser Phe His Asn Gly Thr Leu Leu Thr
                165                 170                 175

Glu Lys Asp Gly Lys Leu Pro Val Arg Asn Thr Met Arg Val Pro Leu
            180                 185                 190

Phe Asn Asn Pro Val Pro Ser Val Met Lys Met Leu Ser Pro Glu Arg
        195                 200                 205

Leu Phe Leu Leu Gly Asp Pro Arg Thr Asn Gln Asn Pro Ala Ile Leu
    210                 215                 220

Ser Phe Ala Ile Leu Phe Leu Arg Trp His Asn Thr Leu Ala Gln Arg
225                 230                 235                 240

Ile Lys Arg Val His Pro Asp Trp Ser Asp Glu Asp Ile Tyr Gln Arg
                245                 250                 255

Ala Arg His Thr Val Ile Ala Ser Leu Gln Asn Val Ile Val Tyr Glu
            260                 265                 270

Tyr Leu Pro Ala Phe Leu Gly Thr Ser Leu Pro Pro Tyr Glu Gly Tyr
        275                 280                 285

Lys Gln Asp Ile His Pro Gly Ile Gly His Ile Phe Gln Ala Ala Ala
    290                 295                 300

Phe Arg Phe Gly His Thr Met Ile Pro Pro Gly Ile Tyr Arg Arg Asp
305                 310                 315                 320

Gly Gln Cys Asn Phe Lys Glu Thr Pro Met Gly Tyr Pro Ala Val Arg
                325                 330                 335

Leu Cys Ser Thr Trp Trp Asp Ser Ser Gly Phe Phe Ala Asp Thr Ser
            340                 345                 350

Val Glu Glu Val Leu Met Gly Leu Ala Ser Gln Ile Ser Glu Arg Glu
        355                 360                 365

Asp Pro Val Leu Cys Ser Asp Val Arg Asp Lys Leu Phe Gly Pro Met
    370                 375                 380

Glu Phe Thr Arg Arg Asp Leu Gly Ala Leu Asn Ile Met Arg Gly Arg
385                 390                 395                 400

Asp Asn Gly Leu Pro Asp Tyr Asn Thr Ala Arg Glu Ser Tyr Gly Leu
                405                 410                 415

Lys Arg His Lys Thr Trp Thr Asp Ile Asn Pro Pro Leu Phe Glu Thr
            420                 425                 430

Gln Pro Glu Leu Leu Asp Met Leu Lys Glu Ala Tyr Asp Asn Lys Leu
        435                 440                 445

Asp Asp Val Asp Val Tyr Val Gly Gly Met Leu Glu Ser Tyr Gly Gln
    450                 455                 460

Pro Gly Glu Phe Phe Thr Ala Val Ile Lys Glu Gln Phe Gln Arg Leu
465                 470                 475                 480

<210> 12
<211> 26
<212> DNA

<213>  Caenorhabditis elegans

<400>  12
tcaagtagtt gcttatgaaa taatgc                                          26


<210>  13
<211>  27
<212>  DNA
<213>  Caenorhabditis elegans

<400>  13
ctagaagtcc tggaacaaag tcatatg                                         27


<210>  14
<211>  23
<212>  DNA
<213>  Caenorhabditis elegans

<400>  14
agtctcccaa tttggctact acg                                             23


<210>  15
<211>  26
<212>  DNA
<213>  Caenorhabditis elegans

<400>  15
acatctgagt gacgaatatg tgtgtc                                          26


<210>  16
<211>  19
<212>  DNA
<213>  Caenorhabditis elegans

<400>  16
cagggtgcca gacgtttgg                                                  19


<210>  17
<211>  19
<212>  DNA
<213>  Caenorhabditis elegans

<400>  17
ccaaacgtct ggcaccctg                                                  19


<210>  18
<211>  36
<212>  DNA
<213>  Heterodera glycines

<400>  18

aattaaccct cactaaaggg atggcgcaac ttcctc 36

<210> 19
<211> 42
<212> DNA
<213> Heterodera glycines

<400> 19
taatacgact cactataggg acgttgtact ccgatcggca ag 42

## Description

The invention relates to assays in nematodes, particularly in plant parasitic nematodes. More specifically, the invention relates to an assay for the detection of nematotoxic compounds which can be ingested by nematodes and including double stranded RNA (dsRNA) molecules with nematicidal activity.

This invention provides a new basis for control of plant parasitic nematodes. The invention is also directed to an assay for detecting compounds for the control of those nematodes that damage crops (crop pests).

The majority of the strategies for protecting against crop pest attack deals with the application of pesticides (*in casu* nematicides). In most cases the pesticides are organic chemical molecules which have to be applied topically at the place where the nematode attacks the organism which needs to be protected. In the case of plants this means spraying the chemical onto the crop, applying during watering of the plants or incorporating it into soil. A preferable process would be for plants to make a biopesticide (i.e. a pesticide *in planta*)*.* To this extent proteinaceous pesticides are advantageous, because they can be expressed in plants through recombinant DNA technology, without the need for specific enzymes or substrates and they are expected to be short-lived in the environment and thus much less damaging to it.

However, there are only a few proteinaceous or peptidergic pesticides known and they are mostly directed to fungi or bacteria. Some of these are toxins, of which the tetanus toxin and the toxins from *Bacillus thuringiensis* form the most well-known and applied group. Some *Bt*-toxins have been reported to be active against plant parasitic nematodes (e.g. WO 99/34926, US 5,831,011, WO 99/06568, and US 5,753,492)

An alternative approach called RNA interference (or RNAi) has been suggested recently (e.g. by Fire et al. 1998, Nature, 391:806-811) who have showed in *C. elegans* that double stranded RNA (dsRNA) can be used to knock-out gene expression when applied to the nematode in the food (Timmons and Fire, 1998, Nature, 395:854) or when the nematode is soaked in such a solution (Tabara et al., 1998, Science, 282:430-431). If essential genes are knocked-out this will result in inhibition of the nematode development or even death of the nematode.

Especially, the proteins or peptides should be active against plant parasitic nematodes that are crop pests. These pests can be subdivided into endoparasites, ectoparasites and ecto-endoparasites of plants. Some are sedentary and others remain mobile as they feed. All use a stylet to pierce plant cell walls and feed by removing plant cell contents before or after plant cell modification. (Symons, P.C. Atkinson, H.J. and Wyss, U. [1994] Annual Review of Phytopathology 32, 235-259). More detail of particular important genera and species, their host ranges and economic importance are defined in standard texts (Luc et al [1990] Plant parasitic nematodes in subtropical and tropical agriculture : Wallingford C.A.B. International ; Evans et al [1993] Plant parasitic nematodes in temperate agriculture Wallingford : CAB International). The genera *Heterodera* and *Globodera* cyst nematodes are important crop pests. They include *H. glycines,* (soybean cyst nematode) *H. schachtii* (beet cyst nematode), *H. avenae* (cereal cyst nematode) and potato cyst nematodes *G. rostochiensis* and *G. pallida.* Root-knot nematodes particularly the genus *Meloidogyne,* damage a wide range of crops. Examples are species *M. javanica, M. hapla, M. arenaria* and *M. incognita.* There are many other economically important nematodes. Both the above groups produce swollen sedentary females as do other economic genera including *Rotylenchulus, Nacobbus,* and *Tylenchulus.* Other economic nematodes remain mobile as adult females and many of these cause damage to a wide range of crops. Examples include species of *Ditylenchus, Radopholous, Pratylenchus, Helicotylenchus* and *Hirschmanniella.* Others do not always enter plants but feed from them as ectoparasites. Examples include *Aphelenchoides, Anguina Criconemoides, Criconema Hemicycliophora, Hemicriconemoides, Paratylenchus* and *Belonolaimus.* Among the ectoparasites the genera *Xiphinema, Longidorus, Paralongidorus, Trichodorus* and *Paratrichodrus* have distinctive importance. They cause damage to crops by their feeding but their economic status as crop pests is often due to roles as vectors of NEPO and TOBRA plant viruses.

Cyst-nematodes as described above are obligate parasites and take several weeks to complete their life-cycle. Both characteristics make them refractory to studies aimed at detecting anti-nematode substances, or in the case of RNAi, to define genes essential for development and plant parasitism. This means that the full range of genetic approaches that have been deployed so incisively with the microbovorous *C. elegans* can not readily be applied to these cyst-nematodes.

Cyst-nematodes only feed following the establishment of a nematode feeding site in the roots of a plant and do not ingest prior to this stage. Further, the small size and well-formed cuticle of a (free-living) second stage juvenile (J₂) of a cyst nematode make microinjection difficult and dependent on specialist equipment, while also soaking of the nematode in a dsRNA preparation does not allow for uptake of dsRNA.

Therefore, there is still need for a feeding assay for plant parasitic nematodes, especially cyst nematodes.

According to an aspect of the invention there is provided a feeding assay for plant parasitic nematodes, characterised in that the assay comprises the following steps: i. induction of feeding in second stage juvenile nematodes (J₂ stage) by treatment of said nematodes with octopamine; ii. feeding them with a test compound; iii. assaying the effect of the compound on the behaviour and development of the nematode.

Preferably, the nematode is a cyst or root-knot nematode and the test compound is a dsRNA.

The assay can measure the ability of the nematode to successfully infect a plant or the effects that are assayed are the subsequent growth and/or sexual fate of the nematode. The nematodes are treated in or with a solution of octopamine in which the concentration of octopamine is brought to a value between 1 and 100 mM, For a cyst nematode it ispreferably higher than 25 mM and most preferably 50mM. For a root-knot nematode it is preferably higher than 1mM and most preferably 10mM. Further part of the invention are compounds which are found active in such an assay, preferably these compounds are double stranded RNA molecules.

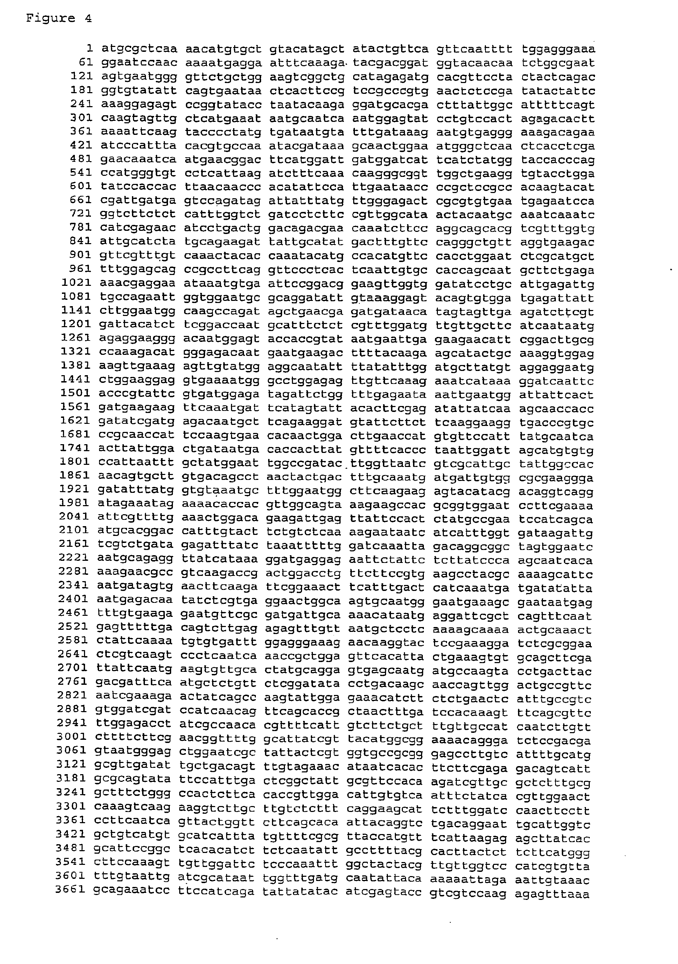

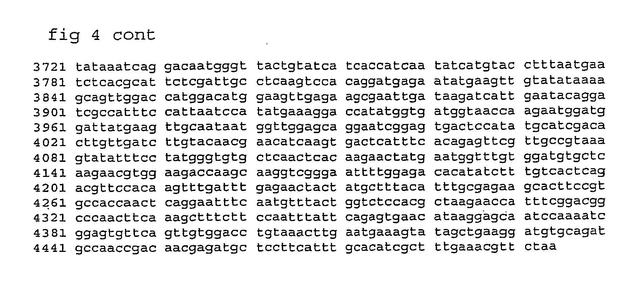

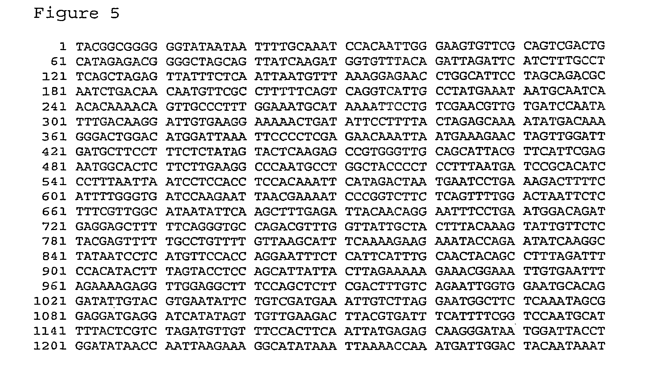

According to a further aspect of the invention there is provided an expression cassette which cassette comprises a nucleic acid sequence which encodes at least part of a gene wherein said nucleic acid sequence is selected from the group consisting of:
i) a nucleic acid sequence represented by the sequence in Figure 4 or Figure 5;
ii) a nucleic acid sequence which hybridizes to the sequence in Figure 4 or 5 and encodes a polypeptide with oxidase activity;
iii) a nucleic acid sequence which is degenerate as a consequence of the genetic code to sequences in (i) and (ii) above,
which cassette is adapted such that both sense and antisense nucleic acid molecules are transcribed from said cassette.

In a preferred embodiment of the invention said hybridisation conditions are stringent.

We have identified homologous genes which are particularly susceptible to RNAi. These genes encode dual oxidases which are involved in crosslinking extracellular matrix (ECM) proteins in the nematode cuticle. The ECM proteins are highly crosslinked by disulphide and by di- tri and isotrityrosine bonds. Recently Edens, W.A. et al. (Journal of Cell Biology 154, 879-891, 2001) identified two dual oxidase (*duox*) genes in *C. elegans* that are expressed in hypodermal cells and play a role in the formation of tyrosine cross-links in the ECM. Duox is a member of the Nox/Duox family of NADPH oxidases that include the phagocyte gp91 *phox* responsible for the generation of reactive oxygen species for bacteriocidal action. These Nox type proteins comprise a 6-transmembrane domain containing two hemes and a cytosolic flavin domain containing an NADPH binding site. The Duox proteins contain, in addition, a cytosolic two EF-hand calcium binding domain, an additional transmembrane-spanning helix and an N-terminal extracellular peroxidase domain.

In a preferred embodiment of the invention said cassette is adapted by the provision of at least two promoter sequences which transcribe sense and antisense nucleic acid molecules from said cassette.

In a further preferred embodiment of the invention said cassette comprises a nucleic acid molecule wherein said molecule comprises a first part linked to a second part wherein said first and second parts are complementary over at least part of their sequence and further wherein transcription of said nucleic acid molecule produces an RNA molecule which forms a double stranded region by complementary base pairing of said first and second parts.

The invention encompasses so called "stem-loop RNA" see Smith et al Nature 407, 319-320. A DNA molecule encoding the stem-loop RNA is constructed in two parts, a first part which is derived from a gene the regulation of which is desired. The second part is provided with a DNA sequence which is complementary to the sequence of the first part. The cassette is typically under the control of a promoter which transcribes the DNA into RNA. The complementary nature of the first and second parts of the RNA molecule results in base pairing over at least part of the length of the RNA molecule to form a double stranded hairpin RNA structure or stem-loop. The first and second parts can be provided with a linker sequence.

In a preferred embodiment of the invention said first and second parts are linked by at least one nucleotide base. In a further preferred embodiment of the invention said first and second parts are linked by 2, 3, 4, 5, 6, 7, 8, 9, or 10 nucleotide bases. In a yet further preferred embodiment of the invention said linker is at least 10 nucleotide bases.

According to a further aspect of the invention there is provided a double stranded RNA molecule wherein said RNA molecule is comprises a nucleic acid sequence selected from the group consisting of:
i) a nucleic acid sequence represented by the sequence in Figure 4 or Figure 5;
ii) a nucleic acid sequence which hybridizes to the sequence in Figure 4 or 5 and encodes a polypeptide with oxidase activity;
iii) a nucleic acid sequence which is degenerate as a consequence of the genetic code to sequences in (i) and (ii) above.

In a preferred embodiment of the invention the length of said dsRNA molecule is between 10 nucleotide bases (nb) and 1000nb. Preferably said dsRNA molecule is 100nb; 200nb; 300nb; 400nb; 500nb; 600nb; 700nb; 800nb; 900nb; or 1000nb in length. More preferably still said dsRNA molecule is at least 1000nb in length.

More preferably still the length of the dsRNA molecule is at least 10nb; 20nb; 30nb; 40nb; 50nb; 60nb; 70nb; 80nb; or 90nb in length.

More preferably still said dsRNA molecule is 21 nb in length.
According to a further aspect of the invention there is provided a vector which includes a nucleic acid cassette according to the invention.

Preferably the nucleic acid in the vector is operably linked to an appropriate promoter or other regulatory elements for transcription in a host plant cell. The vector may be a bi-functional expression vector which functions in multiple hosts. In the example of nucleic acids according to the invention this may contain its native promoter or other regulatory elements.

By "promoter" is meant a nucleotide sequence upstream from the transcriptional initiation site and which contains all the regulatory regions required for transcription. Suitable promoters include constitutive, tissue-specific, inducible, developmental or other promoters for expression in plant cells comprised in plants depending on design. Such promoters include viral, fungal, bacterial, animal and plant-derived promoters capable of functioning in plant cells.

Constitutive promoters include, for example CaMV 35S promoter (Odell et al (1985) Nature 313, 9810-812); rice actin (McElroy et al (1990) Plant Cell 2: 163-171); ubiquitin (Christian et al. (1989) Plant Mol. Biol. 18 (675-689); pEMU (Last et al (1991) Theor Appl. Genet. 81: 581-588); MAS (Velten et al (1984) EMBO J. 3. 2723-2730); ALS promoter (U.S. Application Seriel No. 08/409,297), and the like. Other constitutive promoters include those in U.S. Patent Nos. 5,608,149; 5,608,144; 5,604,121; 5,569,597; 5,466,785; 5,399,680, 5,268,463; and 5,608,142.

Chemical-regulated promoters can be used to modulate the expression of a gene in a plant through the application of an exogenous chemical regulator. Depending upon the objective, the promoter may be a chemical-inducible promoter, where application of the chemical induced gene expression, or a chemical-repressible promoter, where application of the chemical represses gene expression. Chemical-inducible promoters are known in the art and include, but are not limited to, the maize In2-2 promoter, which is activated by benzenesulfonamide herbicide safeners, the maize GST promoter, which is activated by hydrophobic electrophilic compounds that are used as pre-emergent herbicides, and the tobacco PR-1 a promoter, which is activated by salicylic acid. Other chemical-regulated promoters of interest include steroid-responsive promoters (see, for example, the glucocorticoid-inducible promoter in Schena et al (1991) Proc. Natl. Acad. Sci. USA 88: 10421-10425 and McNellie et al. (1998) Plant J. 14(2): 247-257) and tetracycline-inducible and tetracycline-repressible promoters (see, for example, Gatz et al. (1991) Mol. Gen. Genet. 227: 229-237, and US Patent Nos. 5,814,618 and 5,789,156, herein incorporated by reference.

Where enhanced expression in particular tissues is desired, tissue-specific promoters can be utilised. Tissue-specific promoters include those described by Yamamoto et al. (1997) Plant J. 12(2): 255-265; Kawamata et al (1997) Plant Cell Physiol. 38(7): 792-803; Hansen et al (1997) Mol. Gen. Genet. 254(3): 337-343; Russell et al. (1997) Transgenic Res. 6(2): 157-168; Rinehart et al (1996) Plant Physiol. 112(3): 1331-1341; Van Camp et al (1996) Plant Physiol. 112(2): 525-535; Canevascni et al (1996) Plant Physiol. 112(2): 513-524; Yamamoto et al (1994) Plant Cell Physiol. 35(5): 773-778; Lam (1994) Results Probl. Cell Differ. 20: 181-196; Orozco et al (1993) Plant Mol. Biol. 23(6): 1129-1138; Mutsuoka et al (1993) Proc. Natl. Acad. Sci. USA 90(20): 9586-9590; and Guevara-Garcia et al (1993) Plant J. 4(3): 495-50.

"Operably linked" means joined as part of the same nucleic acid molecule, suitably positioned and oriented for transcription to be initiated from the promoter. DNA operably linked to a promoter is "under transcriptional initiation regulation" of the promoter.

In a preferred embodiment the promoter is an inducible promoter or a developmentally regulated promoter.

Particular vectors are nucleic acid constructs which operate as plant vectors. Specific procedures and vectors previously used with wide success upon plants are described by Guerineau and Mullineaux (1993) (Plant transformation and expression vectors. In: Plant Molecular Biology Labfax (Croy RRD ed) Oxford, BIOS Scientific Publishers, pp 121-148). Suitable vectors may include plant viral-derived vectors (see e.g. EP-A-194809).

Vectors may also include selectable genetic marker such as those that confer selectable phenotypes such as resistance to herbicides (e.g. kanamycin, hygromycin, phosphinotricin, chlorsulfuron, methotrexate, gentamycin, spectinomycin, imidazolinones and glyphosate).

According to a further aspect of the invention there is provided a cell transfected or transformed with a cassette or vector according to the invention.

According to a further aspect of the invention there is provided a plant comprising a cell according to the invention.

In a preferred embodiment of the invention there is provided a plant selected from the group consisting of: corn (*Zea mays*)*,* canola (*Brassica napus, Brassica rapa* ssp.), flax (*Linum usitatissimum*), alfalfa (*Medicago sativa*), rice (*Oryza sativa*), rye (*Secale cerale*)*,* sorghum (*Sorghum bicolor, Sorghum vulgare*), sunflower (*Helianthus annus*), wheat (*Tritium aestivum*), soybean (*Glycine max*), tobacco (*Nicotiana tabacum*), potato (*Solanum tuberosum*), peanuts (*Arachis hypogaea*), cotton (*Gossypium hirsutum*), sweet potato (*Iopmoea batatus*), cassava (*Manihot esculenta*), coffee (Cofea spp.), coconut (*Cocos nucifera*), pineapple (*Anana comosus*)*,* citris tree (Citrus spp.) cocoa (*Theobroma cacao*)*,* tea (*Camellia senensis*), banana (*Musa* spp.), avacado (*Persea americana*), fig (*Ficus casica*), guava (*Psidium guajava*), mango (*Mangifer indica*), olive (*Olea europaea*), papaya (*Carica papaya*), cashew (*Anacardium occidentale*), macadamia (*Macadamia intergrifolia*), almond (*Prunus amygdalus*), sugar beets (*Beta vulgaris*), oats, barley, vegetables.

Preferably, plants of the present invention are crop plants (for example, cereals and pulses, maize, wheat, potatoes, tapioca, rice, sorghum, millet, cassava, barley, pea), and other root, tuber or seed crops. Important seed crops are oil-seed rape, sugar beet, maize, sunflower, soybean,sorghum, and flax (linseed).

Horticultural plants to which the present invention may be applied may include lettuce, endive, and vegetable brassicas including cabbage, broccoli, and cauliflower. The present invention may be applied in tobacco, cucurbits, carrot, strawberry, sunflower, tomato, pepper.

Particularly preferred species are those of ornamental plants.

Grain plants that provide seeds of interest include oil-seed plants and leguminous plants. Seeds of interest include grain seeds, such as corn, wheat, barley, rice, sorghum, rye, etc. Oil-seed plants include cotton, soybean, safflower, sunflower, Brassica, maize, alfalfa, palm, coconut, etc. Leguminous plants include beans and peas. Beans include guar, locust bean, fenugreek, soybean, garden beans, cowpea, mungbean, lima bean, fava been, lentils, chickpea, etc.

According to a yet further aspect of the invention there is provided a seed comprising a cell according to the invention.

According to a yet further aspect of the invention there is provided a composition comprising at least one nematicidal dsRNA molecule according to the invention.

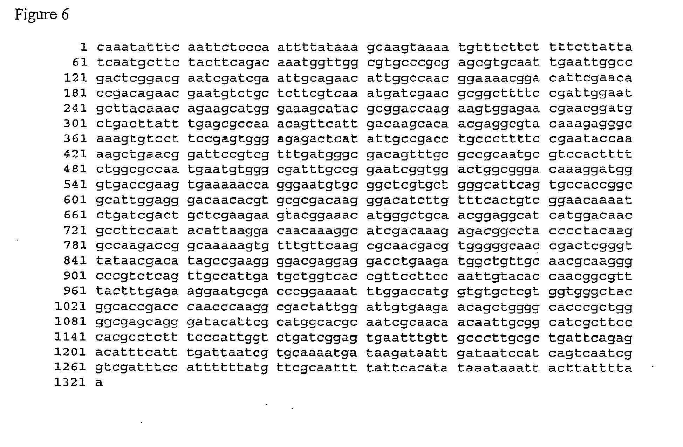

According to a further aspect of the invention there is provided an expression cassette which cassette comprises a nucleic acid sequence which encodes at least part of a gene wherein said nucleic acid sequence is selected from the group consisting of:
i) a nucleic acid sequence represented by the sequence in Figure 6;
ii) a nucleic acid sequence which hybridizes to the sequence in Figure 6 and which encodes a polypeptide with cysteine protease activity;
iii) a nucleic acid sequence which is degenerate as a consequence of the genetic code to sequences in (i) and (ii) above,
which cassette is adapted such that both sense and antisense nucleic acid molecules are transcribed from said cassette.

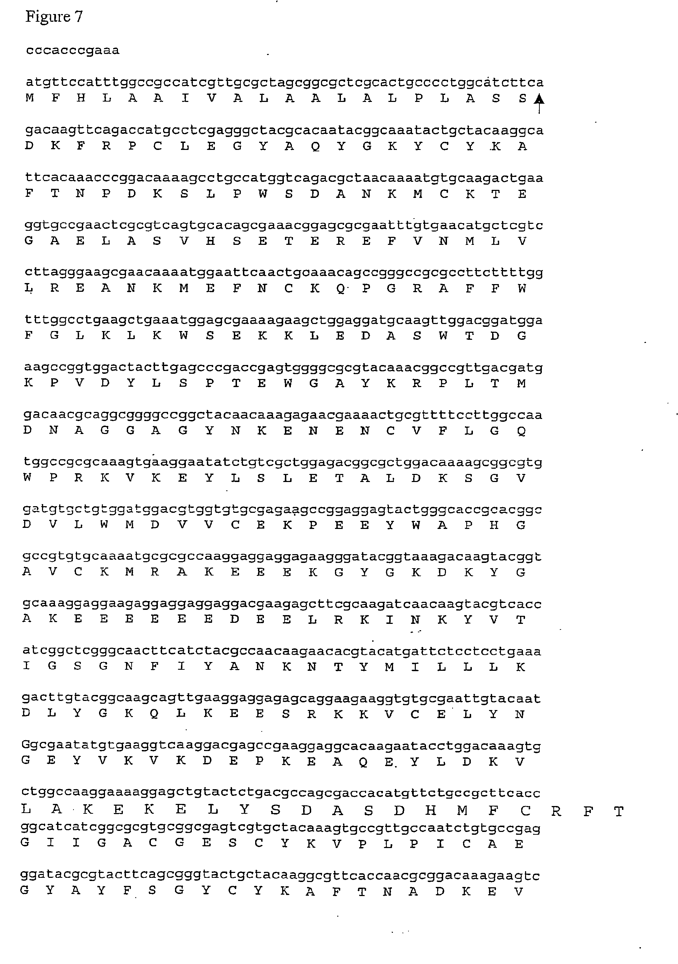

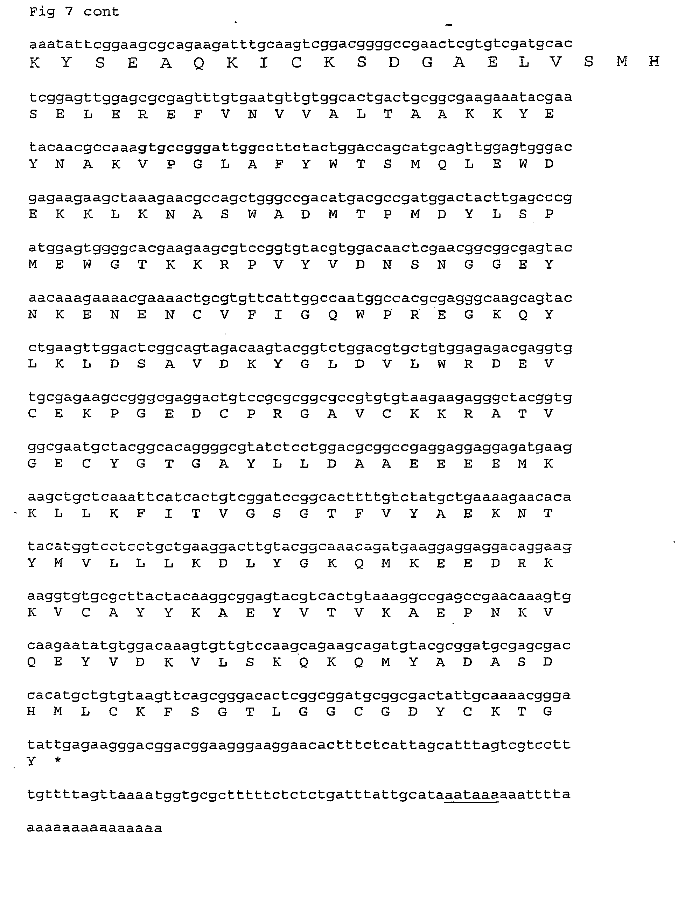

According to a further aspect of the invention there is provided an expression cassette which cassette comprises a nucleic acid sequence which encodes at least part of a gene wherein said nucleic acid sequence is selected from the group consisting of:
i) a nucleic acid sequence represented by the sequence in Figure 7;
ii) a nucleic acid sequence which hybridizes to the sequence in Figure 7;
iii) a nucleic acid sequence which is degenerate as a consequence of the genetic code to sequences in (i) and (ii) above,
which cassette is adapted such that both sense and antisense nucleic acid molecules are transcribed from said cassette.

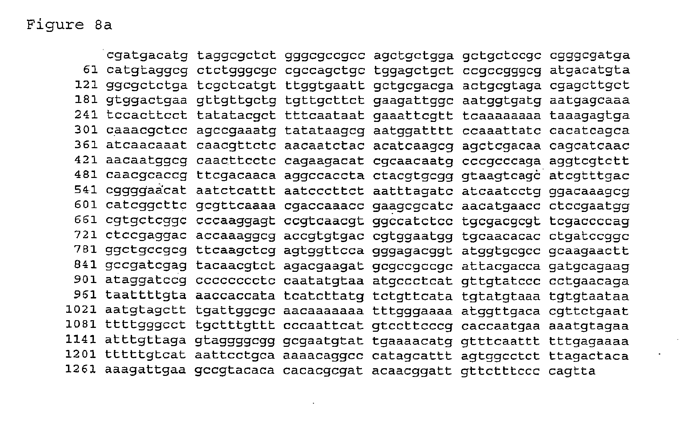

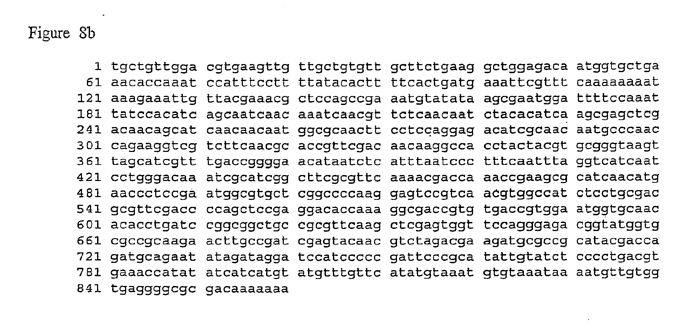

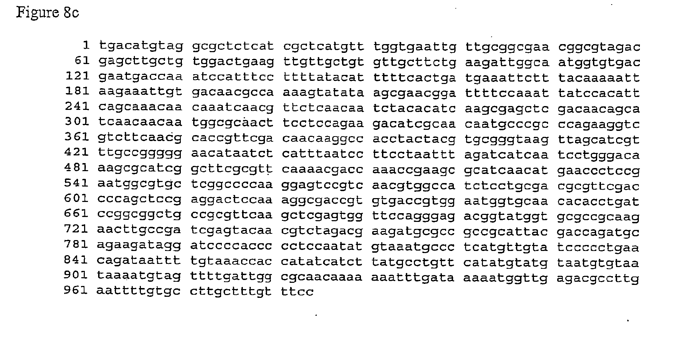

According to a further aspect of the invention there is provided an expression cassette which cassette comprises a nucleic acid sequence which encodes at least part of a gene wherein said nucleic acid sequence is selected from the group consisting of:
i) a nucleic acid sequence represented by the sequence in Figure 8;
ii) a nucleic acid sequence which hybridizes to the sequence in Figure 8 encodes a polypeptide which is a sperm associated polypeptide;
iii) a nucleic acid sequence which is degenerate as a consequence of the genetic code to sequences in (i) and (ii) above,
which cassette is adapted such that both sense and antisense nucleic acid molecules are transcribed from said cassette.

It will be apparent that aspects and preferred embodiments of the invention applicable to the sequences presented in Figure 4 or 5 are equally applicable to those sequences presented in Figures 6, 7 and 8.

In particular, transgenic plants, vectors and compositions which combine two or more dsRNA are also within the scope of the invention. For example a transgenic plant which is transfected with an expression cassette or vector which includes sequences dervived from Figure 4 or 5 with sequences derived from Figure 6 and/or Figure 7 and/or Figure 8. Compositions comprising combinations of dsRNA derived from Figure 4 or 5 with dsRNA derived from Figure 6 and/or Figure 7 and/or Figure 8.

Embodiments of the invention will now be described by examples only and with reference to the following Table and Figures:

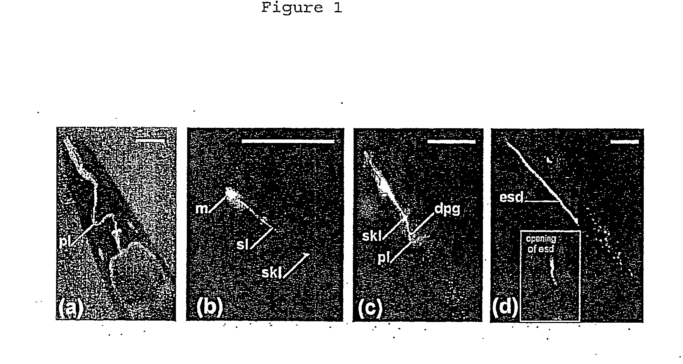

Figure 1 illustrates fluorescence microscopy showing ingestion of FITC. After soaking in 1 mg/ml FITC (a) FITC in the pharangeal lumen (pl) of *C. elegans.* After soaking in 1 mg/ml FITC and 10 mM octopamine, (b) FITC in the lumen of the stylet of *G. pallida;* m, opening of the mouth; sl, stylet lumen; sk, lumen of the stylet knobs (c) FITC in the pharangeal lumen (pl) of *H. glycines;* dpg, duct of the dorsal pharangeal gland. d) FITC in the length of the excretory/secretory duct (esd); inset; the opening of the excretory/secretory duct. No fluorescence was apparent in these structures when nematodes were treated solely with FITC. Bar represents 20 □m;

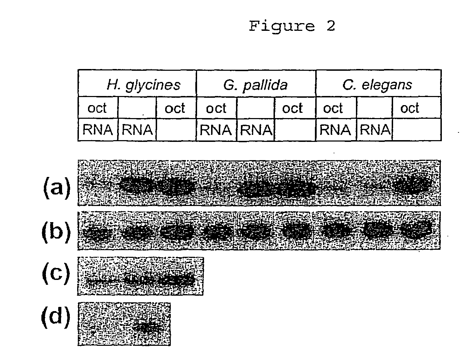

Figure 2 is a virtual northern analysis of transcript abundance. Abundance of (a) a cysteine proteinase (b) actin (c) *hgctl* transcript in pre-parasitic juveniles immediately after soaking. (d) Abundance of MSP in males first treated with dsRNA as juveniles prior to being used to infect plants. oct indicates the presence of octopamine; RNA indicates the presence of dsRNA in the soaking solution;

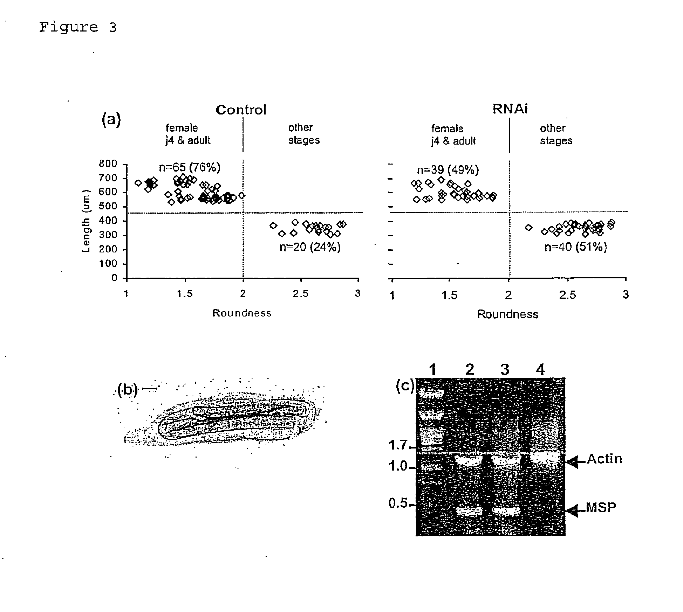

Figure 3 shows growth and sexual dimorphism of *H. glycines.* (a) Filter values for roundness (R), a shape character of (perimeter²) / (4x π x area) which declines to 1 as the saccate condition is achieved and length (L) provide a basis to discriminate between J4 and adult females (R<2; L>469 □m) and other stages (R>2; L≤469□m. At 14 days two groups are apparent, (i) enlarged saccate animals, typically large feeding females (ii) fusiform animals that may include females that are developmentally compromised, J2 animals that have not reached a stage of sexual differentiation and males. The proportions of the two groups is altered following RNAi of cysteine proteinases. (See table 1 for concise comparison of other RNAi treatments) (b) Visual identification of fusiform *H. glycines* (R>2; L≤469□mshowed that nearly all had a vermiform shape inside the J3 cuticle, indicative of males. The remaining animals were developmentally less advanced but had the correct cuticular shape to suggest maleness. Bar = 20 □M. (c) An example of amplification of MSP from individual animals using rtPCR. Lane 1, □ DNA restricted with *pst* I; lane 2, fusiform animal, visually a mature male; lane 3, no visual sign of vermiform shape but male-like cuticular shape; lane 4, cuticular shape less distinctive of a male. A band of the predicted size of MSP was apparent in lanes 2 and 3 but not in lane 4. Amplification of a fragment of the correct size corresponding to actin (c. 2kb) was amplified in all the samples, indicating that the PCR reaction had been successful. It should be noted that less developed males might not provide a PCR fragment representative of MSP;
Figure 4 is the DNA sequence of *duox 1*;
Figure 5 is the DNA sequence of *duox 2*;
Figure 6 is the DNA sequence of a cysteine protease;
Figure 7 is the DNA sequence of the hgctl gene. Primers used to generate a probe for *in situ* hybridisation is underlined in bold.
Figure 8a is the nucleotide sequence of *Globodera rostochiensis* major sperm protein gene *msp-1*; Figure 8b is the nucleotide sequence of *Globodera rostochiensis* major sperm protein gene *msp-2;* and Figure8c is the nucleotide sequence of *Globodera rostochiensis* major sperm protein gene *msp-3.*

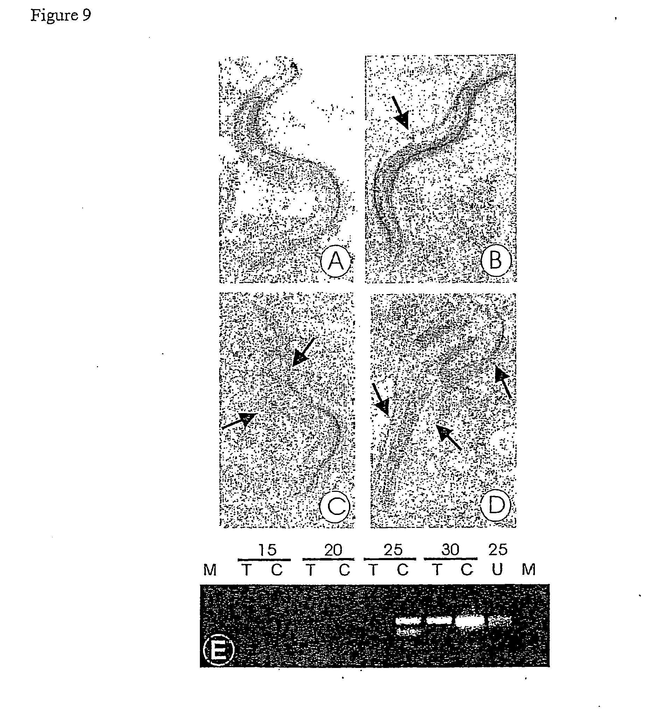

Figure 9 illustrates RNAi phenotype for *duox* in *C. elegans.* (A) control nematode; (B) modest phenotype showing a large blister; (C) dumpy and blistered phenotype; (D) extreme blistered phenotype showing almost complete lack of association of the cuticle to the underlying epidermis. Areas of blistering are indicated by arrows. (E) RT-PCR analysis of the *duox* using mRNA isolated from RNAi-treated (T) and control (C) *C. elegans* L2's, incubated in the appropriate solution for 4 hours. Samples were removed from the PCR after 15, 20, 25 and 30 cycles. There is greater amplification in the control than dsRNA-treated samples indicative of reduced gene expression mediated by RNAi. A control amplification from mRNA isolated from untreated nematodes is shown (U);

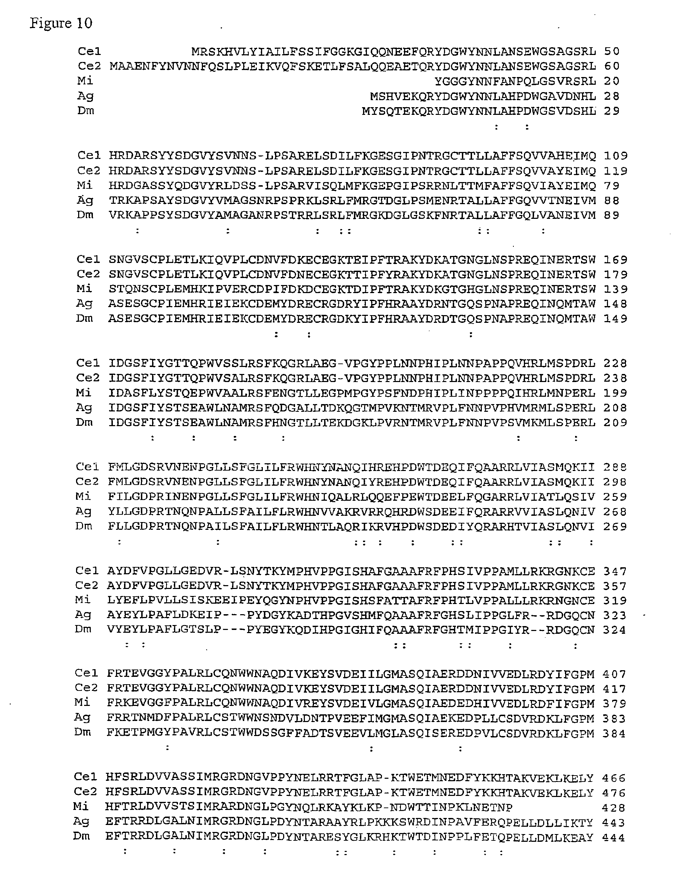

Figure 10. ClustalW alignment of N-terminal peroxidase domains of invertebrate dual oxidase amino acid sequences. Ce1 and Ce2, *C. elegans* DUOX1 (Genbank AAF71303) and DUOX2 (Genbank NP_490684) respectively, Mi, *Meloidogyne incognita* (this work), Ag, *Anopheles gambiae* (Genbank EAA13921), Dm, *Drosophila melanogaster* (Genbank AAF51201). Conserved residues in all sequences are shown by • and similar residues by :, while residues conserved between the three nematode sequences are indicated by o.

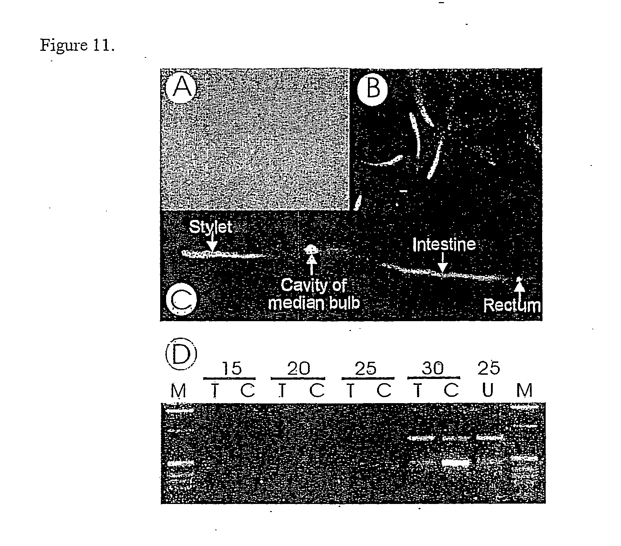

Figure 11. RNAi uptake by non-feeding *M. incognita* J2's. FITC was used to report uptake of material from the incubating solution. (A) no FITC uptake is observed in the absence of octopamine, (B) in the presence of 10mM octopamine efficient uptake of FITC is observed, (C) in some cases very extensive FITC fluorescence is observable throughout the nematode digestive and excretory system. (D) RT-PCR analysis of the *duox* peroxidase using mRNA isolated from RNAi-treated (T) and control (C) *M. incognita* J2's, incubated in the appropriate solution for 4 hours. Samples were removed from the PCR after 15, 20, 25 and 30 cycles. There is greater amplification in the control than dsRNA-treated samples indicative of reduced gene expression mediated by RNAi. A control amplification from mRNA isolated from untreated nematodes is shown (U);

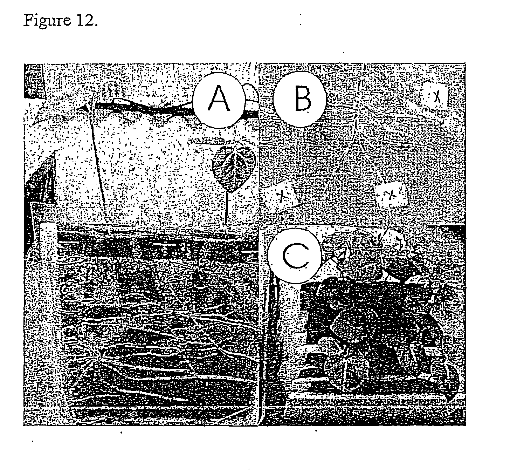

Figure 12 illustration of the pouch system used for aduki bean infection experiments. (A) a single pouch containing 2 plants. (B) expanded view of infected roots with infection points indicated by the filter paper. (C) A series of pouches separated by polystyrene and standing in a tray containing water.

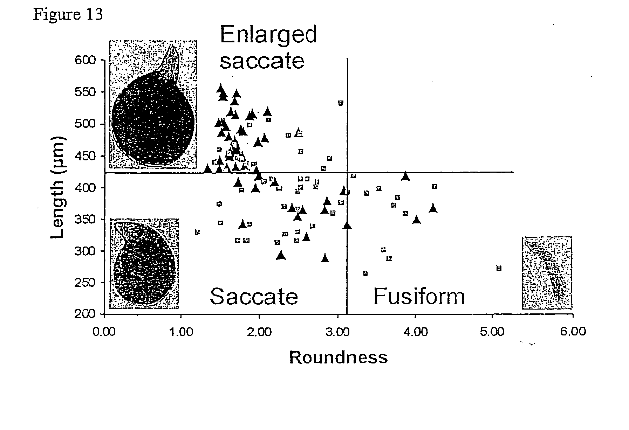

Figure 13. Plot showing size and stage of development of *M. incognita.* The length and roundness filters, indicated by the horizontal and vertical lines, allow assignment of individual nematodes within three classes, fusiform (lower right quadrant), saccate female (lower left quadrant) and enlarged saccate female (upper left quadrant). For the control treatment 55 individuals were measured and are represented by the symbol (▲), similarly 55 individuals from the RNAi treatment were measured and are represented by the symbol (■). Representative nematodes are also shown to scale, indicating the difference in shape and size of the three classes;

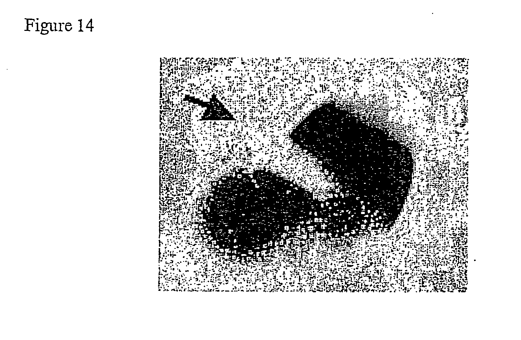

Figure 14. *In situ* hybridisation of *hg-ctl-1* probe to a mature female *H. glycines* showing patterning of the staining. Staining is not apparent in the reproductive system protruding through the damaged body wall *if including above example.*

Table 1 illustrates the number of cyst nematode parasites, mean ± sem for roundness and length of females (J4 and adult) and all other stages. Nematodes were recovered from plants at 14 or 21 days post-infection by RNAi or control treated J2. Comparisons; †, *X*² test for for male / female ratio in RNAi treatments relative to control; t t- test for total parasite burden in RNAi treatment and control; statistical significance, * P<0.05, ** P<0.01, *** P<0.001.

Table 2 Analysis of *M. incognita duox* RNAi experiment. Roots were infected with J2 stage *M. incognita* for both control and RNAi treatments and the number of nematodes were counted at 14 days. A subset of 55 nematodes were then dissected from roots infected with control and RNAi treated nematodes for analysis of roundness and length to classify the growth stage. For analysis at 35 days roots were infected with J2's and nematodes were counted 35 dpi. Egg masses were collected and eggs counted.

### DETAILED DESCRIPTION OF THE INVENTION

Plant parasitic nematodes are important plant pests and cause at least $100 billion annually in global crop losses (J.N. Sasser, W.N. Freckman, in: Vistas on Nematology, J.A. Veech, D.W. Dickson, Eds., Soc. Nematol., Hyattsville, MD, 1987, pp. 7-14). The chemical nematicides are the focus of increasing concern over environmental and toxic risk. Consequently, there has been a recent history of progressive withdrawal from use as a result of changes in governmental regulations.

The assay of the invention is useful for the detection of nematotoxic compounds that exert their toxic effects upon ingestion by the plant parasitic nematode. Especially the assay is useful for the detection of those double stranded RNAs that are capable of inhibiting gene expression in the nematode and thereby affecting the capability of the nematodes to induce syncytium formation in the plant root and/or thereby interfering with the growth and development of the nematode in the sedentary stage.

Novel potential targets for nematode control can be identified *in silico* using a comparative genomics approach based on predicted functions and homology to genes from model organisms which are known to be essential for viability of the organism or crucial for important aspects of its pathogenicity (Lavorgna, G., Boncinelli, E., Wagner, A., and Werner, T. (1998) Detection of potential target genes in silico Trends in Genetics 14(9), 375-376). Such targets can then be validated by functional disruption using RNA interference or by studying knock out mutants of the target gene (WO 00/01846; Bosher, J. M. and Labouesse, M. (2000) RNA interference: genetic wand and genetic watchdog. Nature Cell Biology 2(2), E31-E36; Bird, D. M., Opperman, C. H., Jones, S. J. M., and Baillie, D. L. (1999) The Caenorhabditis elegans genome: A guide in the post genomics age. Annual Review of Phytopathology 37, 247-265). The assay of the invention would be an excellent system for testing if the targets identified by *in silico* experiments would be suitable for development of an *in* vivo anti-nematode approach.

The compounds found as positives in the assay according to the invention can be used in many ways after synthesis. As nematicides they can be administered topically or at the locale of parasites, or pests of plants including the soil and other environments surrounding the plants. Alternatively, they can be produced by transgenic organisms to provide a purifiable substance or an extract for use as above. In addition, they can be produced as a biopesticide to protect the transgenic plant *in planta* from its pathogens, parasites or pests. This can involve constitutive or tissue specific expression of the biopesticide. Expression could also be in response to pathogen, parasite or pest attack and possibly limited to the site of the infection.

A further embodiment of the invention provides a plant which has been transformed with a DNA sequence coding for any of the above mentioned peptides or dsRNA. Such a DNA sequence can be obtained by *de novo* synthesis or by isolating it from a natural source.

In order to be expressed properly the DNA sequence must be operably linked to a promoter. The choice of promoter is dependent on the desired site of expression and also on the desired level of expression and the desired way of regulation of the gene under its control. This is all within ordinary skill.

Unless promoter specificity is particularly preferred strong constitutive promoters can be used which function throughout the whole plant, with as little as possible restriction with respect to developmental patterns. One example of a constitutive promoter for high level expression is the CaMV 35S promoter. Other examples of high-level, light-inducible, promoters are, among others, the ribulose biphosphate carboxylase small subunit (rbcSSU) promoter, the chlorophyll a/b binding protein (Cab) promoter, the chimaeric ferrredoxin/RolD promoter (WO 99/31258) and the like.

In combating root-specific pathogens such as nematodes, root-specific promoters are preferable. Examples of such a promoter are: the RoID, RPL16A. Tub-1, ARSK1, PsMTₐ (WO97/20057), and Atao1 promoter (Møller, S.G. and McPherson, M.J., 1998, The Plant J., 13, 781-791).

Generally, the DNA construct(s) of choice is/are contained in an expression cassette, which comprises at least a promoter and a transcription terminator. It is well known how such elements should be linked in order to function properly and this can be determined without practising inventive skill. A specific method to increase the height of expression of the small peptides of the invention is to include a multitude of coding sequences for these peptides in one gene construct (so-called polyproteins), wherein after transcription the mRNA or the preprotein is processed in such a way that several repeats of the peptide of the invention are generated.

Transformation of plant species is now routine for an impressive number of plant species, including both the *Dicotyledoneae* as well as the *Monocotyledoneae.* In principle any transformation method may be used to introduce chimeric DNA according to the invention into a suitable ancestor cell. Methods may suitably be selected from the calcium/polyethylene glycol method for protoplasts (Krens, F.A. et al., 1982, Nature 296, 72-74; Negrutiu I. et al, June 1987, Plant Mol. Biol. 8, 363-373), electroporation of protoplasts (Shillito R.D. et al., 1985 Bio/Technol. 3, 1099-1102), microinjection into plant material (Crossway A. et al., 1986, Mol. Gen. Genet. 202, 179-185), (DNA or RNA-coated) particle bombardment of various plant material (Klein T.M. et al., 1987, Nature 327, 70), infection with (non-integrative) viruses, in planta *Agrobacterium tumefaciens* mediated gene transfer by infiltration of adult plants or transformation of mature pollen or microspores (EP 0 301 316) and the like. A preferred method according to the invention comprises *Agrobacterium*-mediated DNA transfer. Especially preferred is the use of the so-called binary vector technology as disclosed in EP A 120 516 and U.S. Patent 4,940,838).

Although considered somewhat more recalcitrant towards genetic transformation, monocotyledonous plants are amenable to transformation and fertile transgenic plants can be regenerated from transformed cells or embryos, or other plant material. Presently, preferred methods for transformation of monocots are microprojectile bombardment of embryos, explants or suspension cells, and direct DNA uptake or (tissue) electroporation (Shimamoto, et al, 1989, Nature 338, 274-276). Transgenic maize plants have been obtained by introducing the *Streptomyces hygroscopicus bar*gene, which encodes phosphinothricin acetyltransferase (an enzyme which inactivates the herbicide phosphinothricin), into embryogenic cells of a maize suspension culture by microprojectile bombardment (Gordon-Kamm, 1990, Plant Cell, 2, 603-618). The introduction of genetic material into aleurone protoplasts of other monocot crops such as wheat and barley has been reported (Lee, 1989, Plant Mol. Biol. 13, 21-30). Wheat plants have been regenerated from embryogenic suspension culture by selecting embryogenic callus for the establishment of the embryogenic suspension cultures (Vasil, 1990 Bio/Technol. 8, 429-434). The combination with transformation systems for these crops enables the application of the present invention to monocots.

Monocotyledonous plants, including commercially important crops such as rice, wheat and corn are also amenable to DNA transfer by *Agrobacterium* strains (*vide* WO 94/00977; EP 0 159 418 B1; EP 0 856 060; Gould J, Michael D, Hasegawa O, Ulian EC, Peterson G, Smith RH, (1991) Plant. Physiol. 95, 426-434).

### MATERIALS AND METHODS

### RNAi by feeding in C. elegans

Bacteria HT115 that are RNase III deficient to prevent degradation of dsRNA for RNAi studies were transformed with pPD129.36, a double T7 promoter vector, containing either of the *C. elegans* duox fragments described above. They were plated on NGM agar plates with 50µg/ml ampicillin and 1 mM IPTG (Timmons, et al., Gene 263, 103-112, 2001) and 5-6 one-day adult *C. elegans* (N2) were added and incubated at 20°C for 72 hours. Progeny were scored for phenotype and placed on further plates to score the next generation. An unc-22 fragment cloned in pPD129.36 was used as positive control and displayed a twitching phenotype while OP50 and HT115 bacteria were used as negative controls.

### cDNA library screening

This method provides a basis of using a *C. elegans* cdNA for a duox gene as a basis for finding homologues in a plant parasitic nematode. The method is applied to *M.incognita* as an example of the approach.. A cDNA library of young feeding females of *M. incognita* was kindly provided by Ms Jane Shingles (University of Leeds). Approximately 200 000 plaques were screened in duplicate with ³²P dCTP labeled (Prime It II Random primer labeling kit, Stratagene) a mixed probe. This comprised PCR products (- 650bp) from genomic DNA of *C. elegans* encoding regions of peroxidase and NADPH oxidase domains and generated with primer pairs
CeduoxNf 5'TCAAGTAGTTGCTTATGAAATAATGC3' plus
CeduoxNr 5'CTAGAAGTCCTGGAACAAAGTCATATG C3' and
CeduoxCf 5'AGTCTCCCAATTTGGCTACTACG3' plus
CeduoxCr5'ACATCTGAGTGACGAATATGTGTGTC3'respectively.

Following hybridization for 16 hours at 55°C in 6xSSC, 5xDenhardt's solution and 0.5% SDS, 100µg/ml denatured salmon sperm DNA, washing twice at 55°C in 2x SSC containing 0.1% SDS for 30 minutes, the filters were exposed to autoradiography film (Fuji Super RX film) at -80°C. Secondary and tertiary screening were performed under identical conditions. Plaques were subjected to recombinant pBluescript rescue according to manufacturer's instructions (Stratagene).

### Preparation of dsRNA of a M. incognita duox gene

The cDNA insert was sequenced using an ABI373XL instrument and the coding region (Genbank accession number: XXXXXXX) was translated to give the amino acid sequence of the putative DUOX that was used to perform a PSI-BLAST search. Protein sequence alignments were performed using CLUSTALW. A *Hind*III site 95bp from 5' end and Xhol at 3' end were used to clone a 629 bp fragment into pPD129.36 to give pMiDuox1, which was transformed into HT115 cells for use in *C. elegans* feeding experiments. pMiDuox1 was linearised using *Xho*l and *Xba*l and transcribed *in vitro* (Ampliscribe T7 high yield transcription kit from Cambio.) according to manufacturer's instructions. sense and antisense transcripts were then annealed for 30 minutes at 37°C and analyzed by agarose gel electrophoresis.

### RNAi treatment of M. incognita

The RNAi soaking method was as described for cyst nematodes in example 1. Root balls from infected tomato plant at 8 weeks post infection were washed thoroughly under running tap water. The washed roots were chopped into small pieces (3-4cm) and placed on paper tissue supported on a coarse sieve (250 □m). The sieve was placed inside a funnel over a 50 ml Falcon tube. The funnel was kept under a fine mist of water (25°C) sprayed continuously from mist nozzles. The hatched nematodes were collected in a 50 ml Falcon tube every 24 hours for four days. Approximately 15000 J2 nematodes were soaked in soaking buffer (10mM octopamine in M9 salts (Sigma, Poole, Dorset UK), 1mg/ml FITC in DMF, dsRNA 2µg/µl) for 4 hours at room temperature with occasional flicking in a 1.5 ml microfuge tube. Controls were soaking buffer without dsRNA or without octopamine or both. FITC uptake was analyzed using Ziess axiovert 135-inverted microscope with improvision imaging software and monochromator light source using 520-nm filter. A proportion of dsRNA treated and control animals (no dsRNA) were used for semi-quantitative PCR analysis.

### RNA isolation and semi quantitative PCR

Total RNA was isolated from approximately 12,000 nematodes using RNeasy Mini Kit from Qiagen (Qiagen Ltd). A 2-3µg aliquot of total RNA was reverse transcribed using Super SMART PCR cDNA synthesis kit (BD Bioscience) according to manufacturer's instructions. 3µl aliquot of cDNA reaction mixture was used in each PCR with primers 5' CAGGGTGCCAGACGTTTGG 3' and 5'CCAAACGTCTGGCACCCTG3'. PCR was performed in 100µl reaction containing PCR buffer, 1.5mM MgCl2, 200µMdNTP, and 10pmol/µl primers in a Hybaid Omnigene for 30 cycles of 95°C, 45 sec; 55°C, 30 sec; 72°C for 2 min with a final 5 minute extension at 72°C. The reaction was sampled by removing 10µl aliquots after 15, 20 and 25 cycles for analysis by electrophoresis on 1% agarose gel.

### Infection of plants in pouches

*Phaseolus angularis* (aduki bean) plants were grown in CYG seed growth pouches (Mega International, Minneapolis, USA) and infected with J2 worms as described by Atkinson and Harris (Parasitol. 98: 479-487, 1989). Three to four infection points per pouch were each infected with 15 nematodes. Plants were infected with nematodes treated with soaking buffer containing *duox* dsRNA (RNAi) or without dsRNA (control). The pouches were placed 10 to a tray spaced by 15 mm and immersed in 1cm of water and were grown in a glasshouse at 30°C and 56-60% humidity for 14 or 35 days.

### Staining of nematodes in plant roots and image analysis

The infected roots were collected from the pouches after 14 or 35 days and soaked in 1% bleach for 2 minutes to clear and permeabilise. After rinsing in water they were boiled in acid fuchsin (350mg stain in 1 L of 25% acetic acid) for 2 minutes, were rinsed in water and transferred to the acidified glycerol for examination and dissection²⁵ Atkinson, H.J., et al. (Journal of Nematology 28, 209-215, 1996).Nematodes were dissected from the roots of infected plants by separating them in a 60mm petri dish in acidified glycerol. They were picked at random and dissected out by tearing the root tissue gently using 0.6mm needles. The dissected nematodes were mounted on the glass slide in a drop of acidified glycerol and the measurement of length, area and roundness was carried out using the Image analysis tool kit from LEICA QWin under a Leitz (DMBR) Leica microscope attached to color camera (Kappa F15MCC). At 35 days mature females were picked from the roots using flat bladed tweezers and care was taken to ensure egg masses remained intact. The gelatinous matrix was separated around the eggs using needles and counted on a glass slide from randomly picked mature females. Eggs from 10 females from each treatment were counted and the mean egg number multiplied by total number of females to estimate the total egg count from infected roots.

### Cyst nematodes

Pre-parasitic J2 G. pallida and H. glycines were collected from sterilised cysts as described by Urwin et al., 1995 The Plant Journal, 8, 121-131. G. pallida were hatched at 20 °C and H. glycines at 25 °C. The animals were soaked in 50 mM octopamine made up in M9 salts (43.6 M Na2HPO4, 22 mM KH2PO4, 2.1 mM NaCl, 4.7 mM NH4Cl) together with FITC Isomer I (Sigma, Poole Dorset, UK) 1 mg/ml (stock made up at 20 mg/ml in DMF). The animals were left at RT for 4 h before being collected by brief micro centrifugation and then washed copiously with water to remove any external exogenous dsRNA. Uptake of FITC was viewed by fluorescence using a Leica microscope (model DMRB) with suitable filters. Images were captured with a software package (Quantimet 500c; Leica, U.K) through a either a colour (Kappa CF15 MCC) or black and white (COHU) camera mounted on the microscope. Following treatment, FITC is present in the lumen of the mouth stylet, which is normally used to pierce plant cell walls, and is clearly passed posteriorly to the pharyngeal lumen (Figure 1b,c). The excretory/secretory system of the parasite also shows uptake (Figure 1d). Uptake was typically limited to 15-20% of individuals through the stylet but was often >50% via the excretory/secretory system. Entry by either route may facilitate RNAi as dsRNA has the ability to cross-cellular boundaries (Fire et al. 1998). The inclusion of 3 mM spermidine and 0.05% gelatin in the soaking solution may improve the penetrance of molecules such as dsRNA (Maeda et al., Current Biology, 11. 171-176, 2001) and FITC. The current procedure already provides a basis for applying dsRNA to cyst-nematodes as those animals showing oral uptake of FITC added to the soaking solution can be selected for experiments.

### RNA synthesis

Full length cDNA clones encoding a cysteine proteinase from both H. glycines (hgcp-l) (Urwin et al., Parasitol. 114:605-613, 1997) and its homolog from G. pallida (gpcp-l) (Urwin et al., unpublished isolated by screening G. pallida cDNA library with hgcp-Il were available in the phagemid pBluescript. The coding region of the C. elegans cysteine proteinase gene, gcp-1 (Ray and McKerrow, Mol. Biochem. Parasitol. 51:239-250, 1992) was also available in pBluescript (Urwin et al., Plant J. 8:121-131, 1995). hgctl was isolated by screening a cDNA expression library (Lilley et al., Mol. Bio. Parasitol. 89:195-207, 1997; Urwin et al. Parasitology, 114, 605-613, 1997)) with a monoclonal antibody (N46C10) as described by Sambrook et al., (Molecular Cloning, a laboratory Manual. New York: Cold Spring Harbor Lab. Press, 1989). The antibody was one of a number raised in mouse against total protein extracted from feeding female H. glycines as described by Atkinson et al. (Ann. App. Biol. 112:459-469, 1988). Approximately 440 bp of the major sperm protein (MSP) open reading frame (Novitski et al, J. Nematology, 25, 548-554, 1993) was amplified using the primers:
^{5'}AATTAACCCTCACTAAAGGGATGGCGCAACTTCCTC^{3'}
^{5'}TAATACGACTCACTATAGGGACGTTGTACTCCGATCGGCAAG^{3'}
that incorporate the RNA primer sites T3 and T7 (underlined) respectively. The vectors harbouring hgcp-I and gpcp-I were linearised with Xho I and Eco RI, the vector harbouring gcp-I was linearised with and Sal I and Pst I, the vector harbouring hgctl was linearised with Sac I and Kpn I, when driving transcription from the T3 and T7 promoters respectively. To produce both sense and anti-sense RNA strands in vitro transcription using the T3 and T7 promoters was carried out as specified by the manufacturer (Megascribe, Ambion, Oxfordshire, UK).

### RNAi by soaking

Pre-parasitic J2 G. pallida and H. glycines were collected from sterilised cysts as described by Urwin et al., 1995. G. pallida were hatched at 20 °C and H. glycines at 25 °C. The animals were soaked in 50 mM octopamine made up in M9 salts (43.6 M Na2HPO4, 22 mM KH2PO4, 2.1mM NaCl, 4.7 mM NH4Cl) together with FITC Isomer I (Sigma, Poole Dorset, UK) 1 mg/ml (stock made up at 20 mg/ml in DMF) and dsRNA commonly between 2 and 5 mg/ml. The animals were left at RT for 4 h before being collected by brief micro centrifugation and then washed copiously with water to remove any external exogenous dsRNA. Uptake of FITC was viewed by fluorescence using a Leica microscope (model DMRB) with suitable filters. Images were captured with a software package (Quantimet 500c; Leica, U.K) through a either a colour (Kappa CF15 MCC) or black and white (COHU) camera mounted on the microscope.

### Analysis of transcript abundance

Pre-parasitic J2 animals showing stimulated ingestion by FITC uptake were collected to analyse transcript abundance. PolyA+ RNA was isolated from J2 animals using a QuickPrep Micro mRNA purification kit (Amersham Pharmacia, Bucks, UK). Transcript abundance was determined by virtual northern analysis that utilises SMART PCR cDNA synthesis technology (Clontech, Hampshire, UK). Briefly a modified oligonucleotide (dT) primer primes first strand reaction, reverse transcriptase has a terminal transferase activity that adds a few cysteine residues at the 3' end of the first strand cDNA. An oligonucleotide primer with polyG sequence at its 3' end hybrides to the polyC sequence and acts as an extended template for reverse transcriptase. The oligonucleotide sequences at the 3' and 5' ends are then used to amplify cDNA by IdPCR. The cDNA can then be size fractionated by agarose electrophoresis, transferred to an immobilised support and probed with a suitably radiolabelled-DNA sequence by standard protocols (eg. Sambrook et al, 1998).

### Observation of phenotypes

J2 animals were used to infect plants grown in pouches as described by Atkinson and Harris (Parasitol. 98:479-487, 1989). Animals were collected from the roots after 14 or 21 dpi and studied by image analysis (Atkinson, H.J., et al. (Journal of Nematology 28, 209-215, 1996)). Presumptive males were subject to rtPCR using an ABgene kit (ABgene, Surry, UK). Individual animals were first dissected from root material and ground in a microcentrifuge tube, Hybaid Recovery amplification reagent was used as described by the manufacturer (Hybaid, Middlesex, UK). Primers were designed against the published sequence of MSP (Novitski et al., J. Nematol. 25:548-554, 1993) with the sequence ^{5'}atggcgcaacttcttcc^{3'} and ^{5'}acgttgtactcgatcggcaag^{3'}. As a control in rtPCR work primers were designed to amplify an actin from G.pallida, ^{5'}agtacccgattgagcacggc^{3'} and ^{5'}ggcgaatgggtcggcggatgg^{3'}. It was possible to use all the primers together in the same standard rtPCR reaction.

### EXAMPLE 1

We have explored the potential of the new RNAi technique to deliver dsRNA molecules targeted against three very different cyst-nematode genes. First we targeted cysteine proteinases, as their inhibition by specific protein inhibitors as transgenes in plant hosts affects growth of established parasites. J₂ of H. glycines, G. pallida and mature C. elegans were treated for 4h with combinations of dsRNA, corresponding to a cysteine proteinase from each species (hgcp-I, gpcp-I or gcp-I respectively) with or without 50 µm octopamine, FITC was included as a reporter of uptake. The relative transcript abundance immediately after treatment was determined by virtual northern analysis. Both H. glycines and G. pallida showed reduced cysteine proteinase transcript abundance but only when octopamine was present with the dsRNA (Figure 2a). As expected, the neurohumor was not required for C. elegans to show the effect because the animal readily swallows the medium in which it is bathed. Stripping the northern blot and re-probing it with an actin probe confirmed equal loading of sample cDNA (Figure 2b).

### EXAMPLE 2

The second gene that was selected was a novel H. glycines gene (hgctl) of unknown function. We chose it to explore the potential of RNAi to help define which genes of unknown function must be expressed for normal parasite development. This possibility is explored later using J₂ that had received RNAi treatments to challenge host plants. hgctl was isolated following monoclonal antibody (MAb) screening of a cDNA expression library. The MAb is one of many hundreds originally obtained using a shotgun approach with immunogens of homogenised J₂ animals. hgctl is expressed most abundantly by adult female cyst nematodes during their period of rapid growth. It encodes a protein of 77 kDa with two C-type lectin (ctl) domains that are functional when expressed in E. coli. Its two lectin domains provide sequence homology with a cobra venom coagulation factor, macrophage mannose receptor and proteoglycan core proteins such as aggrecan. C. elegans has a number genes that are predicted to contain C-type lectin domains but none have high homology to hgctl (Lilley in press). RNAi suppressed its transcript abundance in J₂ (Figure 2d).

### EXAMPLE 3

Another example of transcripts we targeted for RNAi are from the 5 similar genes of major sperm protein (MSP) of Globodera. They were chosen for a number of reasons. Any RNAi effect on these male-specific transcripts should not influence development of females. Secondly the gene family is first expressed in male sperm more than 10 days after establishment of the J₂ as a parasite. Nematode sperm in which MSP is compromised are are unlikely to be functional. MSP comprises 10-15% of total sperm protein and is involved in pseudopod formation in these non-flagellate sperm. It probably replaces the function of actin which contributes only 0.02% of sperm protein to nematode sperm (L.Hernault, S.W., 1197 Chapter 11 Spermatogenesis, in C. elegans II Ed Riddle D.L. et al., Cold Spring Harbor Laboratory Press. ISBN 0-87969-488-2 (Finally male sterility by dsRNA has potential for control of amphimicitic plant parasitic nematodes such as cyst nematodes. MSPs are highly conserved proteins and so it follows that demonstration of an RNAi effect against one provides an example that extends to all. Laboratory sterilisation of males by irradiation followed by their release is used for control of some insects (Rhode et al., 1971 Journal of Economic Entomology, 64, 708).

A pool of recovered male nematodes at 15 days post-infection (dpi) that had been treated with dsRNA as J₂ did show a suppression of MSP transcript abundance (Figure 2d). Therefore an RNAi effect can be achieved for a cyst-nematode gene that is first expressed several days after dsRNA treatment. This establishes a potential to treat J₂ and then study those genes expressed when they become established parasites. In C. elegans, RNAi effects can persist throughout an entire life cycle of the progeny of the injected animals (Kuwabara and Coulson, Parasitol. Today 16:347-349, 2000).

The consequence on subsequent plant parasitism of RNAi was studied for each of the three genes targeted at the J₂ stage. Pre-parasitic juveniles of H. glycines and G. pallida were again stimulated to ingest and exposed to dsRNA. Only those taking up FITC were used to infect their host soybean or potato plants and to measure their growth as in previous work (Atkinson, H.J., et al. (Journal of Nematology 28, 209-215, 1996). RNAi treatment of cysteine proteinases of both cyst-nematodes and the C-type lectin domain protein of H. glycines had an effect on subsequent development as parasites. After RNAi of cysteine proteinases there was no decline in the number of nematodes established at 14 dpi and normal growth rates were observed. However sexual fate of some individuals was changed. The proportion of animals developing as females declined significantly from the expected value of 75% on controls to 50% (P<0.001) after RNAi of the cysteine proteinase (Figure 3a; Table 1). The proportion of female G. pallida also fell from 77% in control population to 56% (P<0.01) for those animals cysteine proteinases of which were targeted by RNAi (Table 1). Males have a distinct appearance that allowed visual identification to confirm that filter values used in image analysis correctly defined the sex of an animal (Figure 3b). In addition rtPCR was used to detect major sperm protein (MSP) in individual animals some of which were not as advanced in development as the animal shown in figure 3b (Figure 3c). This combination of methods used to study phenotype gives confidence in defining the sex of an animal but cannot be considered absolute, some developmentally compromised females or juveniles may be included in the group. In a further study involving RNAi of cysteine proteinases the number of animals recovered 21 dpi from plants showed a reduction of 40 % (P<0.05) (Table 1). This reflects the larger proportion of males in the RNAi treated population that by 21 days had left the plant. At both 14 and 21 dpi there no appreciable difference in the size of the females treated with dsRNA corresponding to cysteine proteinases as juveniles and that of the control females. However the measurement of outline area would enable such an effect to be detected following RNAi directed at other targets that do have this phenotypic consequence. dsRNA directed at transcript of hgctl did not affect the sexual development of the animals. However at 14 dpi 41% fewer (P<0.01) animals that had been targeted by RNAi were recovered from plants relative to the controls (Table 1). This would suggest that RNAi treatment of the C-type lectin domain, in comparison to cysteine proteinases compromised successful parasitism earlier in the process of invasion or establishment. The results establish that RNAi can distinguish between different consequences for successful parasitism arising from inhibition of different genes. RNAi targeting of MSP had no affect on either parasite growth or sexual fate (Table 1). As expected there is no evidence that RNAi targeted at MSP influences development of parasites to adults but loss of major sperm protein will compromise the fertility of males. MSP comprises 10-15% of total sperm protein and is involved in pseudopod formation in these non-flagellate sperm. It probably replaces the function of actin which contributes only 0.02% of sperm protein to nematode sperm (L.Hernault, S.W. , 1197 Chapter 11 Spermatogenesis, in C. elegans II Ed Riddle D.L. et al., Cold Spring Harbor Laboratory Press. ISBN 0-87969-488-2.

**Table 1 Table altered to improve layout etc**

| **RNAi Target** | ***Species*** | **dpi** | **Number of parasites** | | | |
|---|---|---|---|---|---|---|
| | | | **Female** | | **Others** | |
| | | | **(J4 & adult)** | | **(J2,J3 & males)** | |
| | | | **RNAi** | **Control** | **RNAi** | **Control** |
| Cysteine proteinase | H. glycines | 14 | 65†*** | 39†*** | 20†*** | 40†*** |
| Cysteine proteinase | H. glycines | 21 | 49‡* | | 27‡* | |
| Cysteine proteinase | G. pallida | 14 | 48†** | 61†** | 37‡** | 18†** |
| C-type lectin | H. glycines | 14 | 32†** | 54†** | 14†** | 24†** |
| MSP | H. glycines | 14 | 41 | 34 | 19 | 20 |

### EXAMPLE 4

*In situ* hybridisation was carried out using single-stranded DNA probes as described by deBoer et al., 1998 (J. Nematology, 30, 309-312) to determine the sites of Hg-ctl-1 expression. An intense hybridisation signal was observed when J3, J4 and adult female stages were treated with an anti-sense probe specific for a 291 bp region of the *hg-ctl-1* gene (from residues 942 to 1233 in Figure 7). In contrast, hybridisation was not detected in J2 nematodes. This is consistent with a stage dependent transcript abundance detected by virtual northerns. Hybridisation was apparent throughout most of the nematode body but was consistently absent from the head and neck region. Dissection of the nematodes revealed that the staining was restricted to the outer body wall layer and was not present in the reproductive system or intestinal tissue. A mosaic-like pattern of staining was occasionally visible within the body wall as can be seen in (Fig. 14). The observed pattern of gene expression suggests that Hg-ctl-1 is secreted to the surface coat of the nematode. It is most abundantly expressed in the body surface regions that show rapid expansion of the body circumference in saccate females. It is not abundantly expressed in the neck or vulva region where such expansion is more limited. Its lower abundance in mature females suggests it does not play a major role in protection of the female when exposed on the root surface

### EXAMPLE 5

The two dual oxidase genes of *C. elegans, duox-1* and *duox-2,* are 88.8% identical at the nucleotide sequence level. Conserved regions from the N-terminal peroxidase and C-terminal NADPH oxidase encoding regions were used in RNAi feeding experiments and confirm the results of Edens, W.A. et al. (Journal of Cell Biology 154, 879-891, 2001).The majority of nematodes (>95%) showed one or more large blisters and most (>85%) were of a dumpy appearance (Figure 9). The posterior ends were often distended with unlaid eggs and dumpy phenotypes never released eggs. Of eggs that were released the vast majority (>95%) failed to hatch. Predominantly the nematodes were relatively immobile or showed paralysis. Semi-quantitative RT-PCR reveals lower levels of *duox* transcript in treated compared with control samples (Figure 9). Global expression studies have revealed cyclic expression of *duox* genes during embryogenesis and at 36h, consistent with a role in formation of new cuticle. The highly disruptive RNAi phenotype for duox in *C. elegans* led us to search for homologues in plant nematodes.

Using a mixed N- and C-terminal probe from the *C. elegans duox1* we screened a *Meloidogyne incognita* cDNA library and identified a clone with an insert of 723bp that showed 65% nucleotide sequence identity to part of the N-terminal peroxidase domain of *duox1.* Subsequent PCR amplification from the cDNA library has extended the length of sequence to 1285bp. That this region is duox-derived rather than from a peroxidase was supported by the amino acid sequence identity between *C. elegans* DUOX1 and the putative *M. incognita* DUOX homologue, which was 67% compared with only 30% identity with other *C. elegans* haem peroxidases (Figure 10). Southern blot analysis has revealed the likely presence of three *duox-*related genes in *M. incognita* (data not shown).

### EXAMPLE 6

We performed a series of RNAi experiments using a 629bp region of the putative *M. incognita duox* gene. There was no RNAi phenotype observed by feeding of *C. elegans*, indicating that 65% nucleotide identity is insufficient of elicit an RNAi response. Experiments were then performed with J2 stage *M. incognita* placed in solutions containing various combinations of dsRNA, FITC and 10mM octopamine and incubated for four hours. FITC uptake was observed only in the presence of octopamine with the majority of nematodes (>95%) showing significant fluorescence often distributed along their length (Figure 11). This efficient dye uptake contrasts with the limited uptake observed for cyst nematodes (c/f Figure 1b-d with Figure 11c)where the presence of the dye allows selection of nematodes for further study. Root knot nematodes may provide a better target for RNAi studies due to this efficient uptake that removes the need to select affected nematodes. Extensive distribution of dsRNA, by analogy with *C. elegans,* would be expected to elicit an efficient amplification response leading to systemic RNAi.

Following dsRNA treatment, semi-quantitative RT-PCR was used to analyse the relative levels of *duox* transcript in control and dsRNA-treated samples. The level of mRNA was significantly lower in duox-treated compared with control treated J2's (Figure 11) as shown previously for *C. elegans.* Nematodes were also used to infect aduki beans (*Phaseolus angularisin*) in a soil-free pouch system, which allows the level and synchrony of infection to be controlled and the infection sites to be easily monitored and subsequently dissected. The system used is shown in Figure 12 was also used for cyst nematodes with soybean or potato planlets in example 3.

For analysis at 14 dpi, roots were infected with control or *duox* dsRNA-treated J2 *M*. *incognita.* The number of galls were counted to determine the level of infection and this was the same for control and test sample indicating that *duox* is not involved in the migration or infection processes. Roots were stained with acid fuchsin to detect nematodes. By 14 days the numbers of establish nematodes were significantly different with 413 for the control treatment but only 165 for the duox-dsRNA treatment, a 60% reduction. The reduced number of nematodes could be due to premature death resulting from failure to develop or to an increase in the number of males that then leave the root although, as observed for cyst nematodes¹², males should still be present at 14 days.

Image analysis was used to measure roundness (R), area (A) and length (L) for 55 randomly dissected nematodes from each treatment (Table 2). We have previously shown that the ratio of roundness to length allows classification of *M. incognita* into three morphological classes, fusiform (J2, J3, J4, male), saccate females and gravid females (Atkinson, H.J., et al., Journal of Nematology 28, 209-215, 1996). Such measurements provide information on the relative growth rates and stages of development of nematodes within the population. Filter values for R and L of *M. incognita* individuals can be applied to separate the population into three classes; fusiform (J2, J3, J4 and male) R>3.06 and L<422□m; saccate (young female) R<3.06 and L<422□m; enlarged saccate (gravid female) R<3.06 and L>422□m²⁵ (Atkinson, H.J., et al., Journal of Nematology 28, 209-215, 1996). Data for 55 individuals from both control treated (black squares) and *duox* dsRNA-treated nematodes (open circles) are shown in Figure 13. The numbers of fusiform and saccate females are significantly higher for the duox-dsRNA treated population than for the control, while the number of enlarged females is significantly greater in the control than the dsRNA treated population (Table 2). Chi square analysis reveals a very highly significant difference in proportions of the three categories of nematodes between the two treatments (P< 0.001). In addition, the size of nematodes at 14 days post infection was much smaller after RNAi treatment establishing a reduced growth rate (Table 2).

**Table 2.**

| | | **Control** | **RNAi** |
|---|---|---|---|
| **Day 14** | | | |
| Number of nematodes | | 413 | 165 |
| Classification of 55 dissected females | fusiform | 3 | 12 |
| | saccate | 14 | 25 |
| | enlarged | 38 | 18 |
| Mean area (mm²) | | 0.0779 ± 0.0045 | 0.0492±0.0033 |
| Mean length (mm) | | 0.444 ± 0.009 | 266 ± 8 |
| Mean roundness | | 2.03 ± 0.09 | 2.49 ± 0.11 |

| **Day 35** | | | |
|---|---|---|---|
| Number of nematodes | | 312 | 126 |
| Mean egg number per female | | 676 ± 50 | 514 ± 48 |
| Estimated total number of eggs | | 210,912 | 64,764 |

For analysis at 35 dpi, roots were infected with control or *duox* dsRNA-treated J2 *M. incognita.* The 35 dpi analysis involved counting the numbers of nematodes after acid fucshin staining and the eggs as shown in Table 2. The number of control treated nematodes (312) in the roots remained 2.5-fold greater than for *duox* dsRNA treated nematodes (126) indicating that the 60% reduction in nematode numbers was maintained throughout the experimental period. There was a substantial difference in the average number of eggs per female between the two treatments, when ten individuals for each treatment were analysed, with 676 ± 50 per female for the control and 514 ± 48 per female for the *duox* RNAi sample.

### EXAMPLE 7

Egg numbers determined from the numbers of females and average eggs per female provide an important measure of reproductive success. The difference between the values of 210,912 eggs from the control population compared with 64,764 eggs from the treated population (Table 2) represents a 70% reduction in eggs produced. This value can be compared with the level of 70% field resistance Urwin et al 2001, Molecular Breeding 8, 95-101 achieved by transgenic expression of phytocystatins within plant roots to disrupt nematode feeding. The *duox* RNAi effect presented here is much more dramatic as it arises from a single 4 hour pre-infection treatment with dsRNA, and yet it elicits a response that has consequences measurable 35 days later. The observation that a sub-set of nematodes continue to develop following RNAi treatment, but are generally smaller and have fewer eggs, is consistent with an early disruptive RNAi effect. This may lead to arrest of development, an inability of the majority of nematodes to develop further, or to form males, but then release from arrest for the remainder of the nematodes as the RNAi effect decreases. These nematodes may then be competent to increase in size and produce the same number of eggs as seen for the control treatment, but they would be significantly delayed compared with the control nematodes. This hypothesis suggests that exposure of nematodes to appropriate dsRNA molecules during feeding, from the plant, should mediate a prolonged effect on nematode development leading potentially to full resistance.

The molecular consequences of RNAi of Duox remains to be determined, however evidence from *C. elegans* suggests the effect is likely to be on normal cuticle biosynthesis. RNAi effect on *C*. *elegans* yield phenotypes comparable with genetic defects leading to defective collagen and cuticle biosynthesis. This hypothesis was substantiated by Edens et al. (Journal of Cell Biology 154, 879-891, 2001) who used EM to reveal abnormalities of the cuticle including broken and distended struts between the cortical and basal layers, separation of these layers and expansion of the fluid cavity, giving rise to blistering. In addition they used HPLC of acid hydrolysates of wild type and RNAi *C*. *elegans* to demonstrate the presence of di- and trityrosine in wild-type but not in the RNAi animals. Evidence was also presented that the peroxidase domain displays peroxidase activity and the ability to cross-link tyrosine ethyl esters. These observations are consistent with a role for DUOX in cuticle biosynthesis, a process that is extremely important in root knot nematodes not only preceding each moult, but also to allow the rapid expansion of the female body critical for reproductive success.

This study confirms that RNAi studies in plant parasitic nematodes can provide important information on gene function. Our results demonstrate the importance of *duox* in *M*. *incognita,* probably related to ECM development, and identify these genes as potential targets for the development of transgenic resistance strategies. In particular we have shown that a single pre-infection dsRNA treatment results in RNAi effects whose consequences on nematode development are observed as a 70% reduction in egg numbers at 35 days after treatment.

The use of highly selective dsRNA molecules targeted at nematode ECM processes without production of foreign proteins may prove more acceptable to critics of GM approaches to pest control. It will be important to investigate the potential for delivery of dsRNA molecules from plant cells to feeding nematodes. Plants display post-transcriptional gene silencing that operates in the same manner as RNAi in nematodes with the dsRNA degraded to siRNAs of around 21 nucleotides. This seems likely to represent a natrual defence strategy against viruses as well as a mechanism for normal gene regulation. Plant constructs have been generated to allow expression of dsRNA molecules to silence genes within plants to explore gene function. Control of nematodes presents additional challenges as it may require production in the plant cell of long dsRNAs for uptake by the nematode, for subsequent processing to elicit an RNAi response. The efficacy of different dsRNA constructs including siRNAs should be explored to determine whether it is necessary to overcome plant cell processing of nematode targeted dsRNA.

## Claims

1. A transgenic soybean plant cell which provides resistance to infestation by an obligate nematode wherein said cell is in the roots of a transgenic soybean plant and comprises an expression cassette for producing double stranded (ds)RNA which is ingested by a cyst or root-knot nematode after establishment of a nematode feeding site in said roots and wherein said resistance provides a reduction in the number of nematode eggs that are produced.

2. A soybean plant of claim 1 wherein said dsRNA changes the sexual fate of nematodes that ingest said dsRNA.

3. A soybean plant of claim 1 wherein said dsRNA compromises the fertility of male nematodes that ingest said dsRNA.

4. A soybean plant of claim 1 wherein said dsRNA has the effect of reducing the number of gravid female nematodes infesting said plant.

5. A soybean plant of any of claims 1 to 4 wherein said dsRNA is targeted to a major sperm protein.

6. A method for controlling obligate nematode infestation of soybean plants comprising providing in root cells of a soybean plant an expression cassette for producing dsRNA which is ingested by a cyst or root-knot nematode after establishment of a nematode feeding site in said roots and wherein said resistance provides a reduction in the number of nematode eggs that are produced.
